(19) Europäisches Patentamt
European Patent Office
Office européen des brevets

(11) EP 1 776 103 B1

(12) FASCICULE DE BREVET EUROPEEN

(45) Date de publication et mention de la délivrance du brevet:
**08.07.2009 Bulletin 2009/28**

(21) Numéro de dépôt: **05790998.8**

(22) Date de dépôt: **13.07.2005**

(51) Int Cl.:
***A61K 31/295*** *(2006.01)* ***C07F 15/00*** *(2006.01)*
***A61P 35/00*** *(2006.01)*

(86) Numéro de dépôt international:
**PCT/FR2005/001814**

(87) Numéro de publication internationale:
**WO 2006/016069 (16.02.2006 Gazette 2006/07)**

(54) **COMPLEXES DE RUTHENIUM POUR LE TRAITEMENT DE CANCERS**

RUTHENIUMKOMPLEXE ZUR KREBSBEHANDLUNG

RUTHENIUM COMPLEXES FOR TREATING CANCERS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **13.07.2004 FR 0407819**

(43) Date de publication de la demande:
**25.04.2007 Bulletin 2007/17**

(73) Titulaire: **Université de Strasbourg**
**67000 Strasbourg (FR)**

(72) Inventeurs:
- **PFEFFER, Michel**
  **F-67100 Strasbourg (FR)**
- **SIRLIN, Claude**
  **F-67800 Bischeim (FR)**
- **LOEFFLER, Jean-Philippe**
  **F-67370 Berstett (FR)**
- **GAIDDON, Christian**
  **F-67000 Strasbourg (FR)**
- **BISCHOFF, Pierre**
  **F-68500 Guebwiller (FR)**
- **JEANNEQUIN, Pierre**
  **F-67000 Strasbourg (FR)**

(74) Mandataire: **Tezier Herman, Béatrice et al**
**Cabinet Becker & Associés**
**25, rue Louis Le Grand**
**75002 Paris (FR)**

(56) Documents cités:
**WO-A-00/56743** **WO-A-97/36595**

- **RYABOV A D ET AL: "New synthesis and new bio-application of cyclometalated ruthenium(II) complexes for fast mediated electron transfer with peroxidase and glucose oxidase." INORGANIC CHEMISTRY. 3 DEC 2001, vol. 40, no. 25, 3 décembre 2001 (2001-12-03), pages 6529-6532, XP002317880 ISSN: 0020-1669**
- **DATABASE CA [Online] CHEMICAL ABSTRACTS SERVICE, COLUMBUS, OHIO, US; DAVYDOVA, M. E. ET AL CLARK, ALEX M. ET AL: "Stability and catalytic properties of glucose oxidase from Penicillium funiculosum G-15 Electrophilic Substitution Reactions at the Phenyl Ring of the Chelated 2-(2'-Pyridyl)phenyl Ligand Bound to Ruthenium(II) or Osmium(II)" XP002359467 extrait de STN Database accession no. 2003: 210594 & VESTNIK MOSKOVSKOGO UNIVERSITETA, SERIYA 2: KHIMIYA , 43(6), 366-370 CODEN: VMUKA5; ISSN: 0579-9384 ORGANOMETALLICS , 18(15), 2813-2820 CODEN: ORGND7; ISSN: 0276-7333, 1999,**
- **LE LAGADEC, RONAN ET AL: "Cyclometalated N,N-dimethylbenzylamine ruthenium(II) complexes [Ru(C6HR1R2R3-o-CH2NMe2)(bpy)(RCN)2]PF6 for bioapplications: synthesis, characterization, crystal structures, redox properties, and reactivity toward PQQ-dependent glucose dehydrogenase" JOURNAL OF ORGANOMETALLIC CHEMISTRY , 689(25), 4820-4832 CODEN: JORCAI; ISSN: 0022-328X, 2004, XP002317879**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



(Cont. page suivante)

- RYABOV, ALEXANDER D. ET AL: "Redox Mediation and Photomechanical Oscillations Involving Photosensitive Cyclometalated Ru(II) Complexes, Glucose Oxidase, and Peroxidase" ANALYTICAL CHEMISTRY , 77(4), 1132-1139 CODEN: ANCHAM; ISSN: 0003-2700, 2005, XP002359422
- CLARK, ALEX M. ET AL: "Electrophilic Substitution Reactions at the Phenyl Ring of the Chelated 2-(2'-Pyridyl)phenyl Ligand Bound to Ruthenium(II) or Osmium(II)" ORGANOMETALLICS , 18(15), 2813-2820 CODEN: ORGND7; ISSN: 0276-7333, 1999, XP002359423
- RITLENG, VINCENT ET AL: "Reaction between Ethylene and Cycloruthenated Tertiary Amines: Stoichiometric Olefin Arylation and Stereospecific One-Carbon-Atom Insertion" ORGANOMETALLICS , 22(2), 347-354 CODEN: ORGND7; ISSN: 0276-7333, 2003, XP002317878
- PEREZ, JULIO ET AL: "Synthesis and Structure of the First Ruthenated Benzodiazepines" ORGANOMETALLICS , 21(24), 5437-5438 CODEN: ORGND7; ISSN: 0276-7333, 2002, XP002317881 cité dans la demande
- FLOWER, KEVIN R. ET AL: "Synthesis and Characterization of Cycloruthenated 2-(Phenylimino)phenyls: A Useful Probe for the Elucidation of the Tautomeric Process in 2-Hydroxyphenyl-Schiff Bases" ORGANOMETALLICS , 21(6), 1184-1189 CODEN: ORGND7; ISSN: 0276-7333, 2002, XP002317882 cité dans la demande
- DATABASE CA [Online] CHEMICAL ABSTRACTS SERVICE, COLUMBUS, OHIO, US; IVANOVA, E. V. ET AL: "Enzyme-substrate interactions in oxidation of (+)- and (-)-[Ru(phpy)(phen)2]PF6 by hydrogen peroxide in the presence of horseradish peroxidase" XP002317883 extrait de STN Database accession no. 2003:210673 & VESTNIK MOSKOVSKOGO UNIVERSITETA, SERIYA 2: KHIMIYA , 43(6), 424-426 CODEN: VMUKA5; ISSN: 0579-9384, 2002,

## Description

La présente invention concerne les compositions pharmaceutiques comprenant des composés du ruthenium ainsi que leurs utilisations dans des méthodes pour le traitement de pathologies prolifératives, en particulier des cancers.

Elle a trait également à de nouveaux composés du ruthénium, ainsi qu'à leur procédé de préparation.

Il est connu que les composés métalliques de la mine du platine présentent des activités antitumorales importantes. Le plus connu d'entre eux est le cisplatine qui est couramment utilisé pour le traitement clinique de nombreux cancers. La résistance de certaines cellules cancéreuses et la toxicité intrinsèque du platine font partie des problèmes rencontrés lors de l'utilisation de ce composé. Depuis les années 70, les recherches s'intensifient pour trouver des molécules susceptibles de se substituer au cisplatine et depuis quelques années des composés contenant du ruthénium sont apparus comme pouvant être une alternative intéressante à ceux qui contiennent du platine. Certains complexes du ruthénium ont ainsi déjà été décrits comme pouvant être une alternative lors de traitements anticancéreux (WO 9736595, WO 0056743).

Il existe donc un besoin pour de nouveaux agents anti-cancéreux qui pourraient être une alternative à ceux actuellement utilisés et/ou qui présenteraient des effets secondaires indésirables moindres.

La présente invention propose ainsi des composés du ruthénium qui présentent des propriétés anti-tumorales particulièrement intéressantes. Ces composés sont des composés organométalliques, c'est-à-dire qu'ils contiennent au moins une liaison covalente Carbone-Ruthénium (C-Ru). De plus, cette liaison C-Ru est stabilisée par une liaison intramoléculaire azote-ruthénium (N-Ru), l'azote étant un élément de la partie organique liée au métal par l'atome de carbone, conformément au schéma ci-dessous :

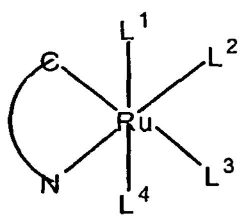

Dans ce type d'arrangement d'atomes, le ruthénium fait donc partie d'une entité cyclique et cette classe de composés est communément appelée par les chimistes de la discipline, la classe des composés cyclométallés. L'entité cyclique contenant le ruthénium est appelée un métallocycle. Dans un métallocycle, le métal est donc lié à la fois à un ligand organique par une liaison covalente carbone-métal et une liaison de type donneur-accepteur (acide-base de Lewis, ou liaison de coordination) azote-métal. L'existence d'un métallocycle dans une molécule organométallique confère à celle-ci des propriétés particulières en terme de réactivité et de stabilité thermodynamique. Divers types de carbones (aromatique, benzylique ou aliphatique) peuvent être métallés et la nature du lien entre l'atome donneur (l'azote) et le carbone peut être modifiée de multiples façons.

Selon un premier aspect, la présente invention a donc pour objet une composition pharmaceutique comprenant, dans un support acceptable sur le plan pharmaceutique, au moins un composé complexe du ruthénium (II) de formule générale (I) ou (II) suivante :

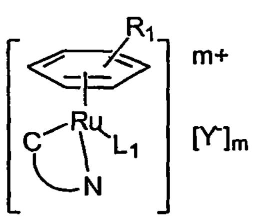

(I)

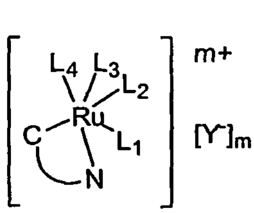

(II)

formule (I) ou (I!) dans laquelle :

$L_1$ $L_2$, $L_3$ et $L_4$, identiques ou différents, représentent soit un ligand donneur de 2 électrons par un atome d'azote,

d'oxygène, de phosphore ou de soufre, soit un atome d'halogène,
R1 représente un atome d'hydrogène ou une ou plusieurs substitutions sur le groupe phényle, choisie parmi un radical (C1-6)alkyle et (C6-18)aryle,
Y est un contre-ion (lorsque m = 1),
m est 0 ou 1,
entre C et N, représentée par une ligne courbe, il existe une succession d'atomes formant, avec les atomes de carbone, d'azote et de ruthénium représentés sur les formules (I) et (II), le métallocycle, qui est constitué de 5 à 8 atomes (incluant les atomes de carbone, d'azote et de ruthénium représentés dans les formules (I) et (II)).

Les composés de l'invention peuvent être sous forme de sels, solvates et/ou de prodrogues pharmaceutiquement acceptables. Les pro-drogues sont des variants des composés de l'invention qui peuvent être transformés *in vivo* en composés de formule (I) ou (II) selon l'invention.

Selon l'invention, le terme "alkyle" désigne un radical hydrocarboné linéaire ou ramifié ayant avantageusement de 1 à 6 atomes de carbone, tel que méthyle, éthyle, propyle, isopropyle, butyle, isobutyle, *tert*-utyle, pentyle, néopentyle, n-hexyle, etc. Les groupes en $C_1$-$C_4$ sont préférés. Les groupes alkyles peuvent être substitués par un groupe aryle tel que défini ci-après, auquel cas on parle de groupe arylalkyle. Des exemples de groupes arylalkyle sont notamment benzyle et phénéthyle.

Les groupes « aryle » sont des systèmes hydrocarbonés aromatiques mono-, bi- ou tri-cycliques, éventuellement interrompus par au moins un hétéroatome (en particulier O, S ou N). Préférentiellement, les groupes aryle incluent les systèmes hydrocarbonés aromatiques monocycliques ou bi-cycliques ayant de 6 à 18 atomes de carbone, encore plus préférentiellement 6 atomes de carbone. On peut citer par exemple les groupes phényle, naphtyle et bi-phényle. Lorsqu'ils sont interrompus par des hétératomes, les groupes aryle incluent les cycles pyridyle, imidazoyle, pyrrolyle et furanyle. Les groupes aryle peuvent éventuellement présenter un ou plusieurs substituants, choisis notamment parmi un atome d'halogène, un groupe alkyle tel que défini ci-dessus, un radical alkoxy (-O-alkyl), thiol, thioether (-S-alkyl), hydroxyl, nitro, cyano et ester (-$CO_2$-alkyl).

Par « halogène », on entend un atome de fluor, de chlore, de brome ou d'iode. Avantageusement, l'atome d'halogène est le chlore.

Les ligands donneurs de deux électrons par un atome d'azote, d'oxygène, de phosphore ou de soufre incluent, par exemple, $H_2O$, di(($C_{1-6}$)alkyl)O, di(($C_{1-6}$)alkyl)S, di(($C_{1-6}$)alkyl)S(O), (($C_{1-6}$)alkyl)$SO_3$, di(($C_{1-6}$)alkyl)C=O, ($C_{1-6}$)alkyl$CO_2^-$. D'autres ligands incluent notamment des ligands nitriles, comme par exemple des ligands de formule ($C_{1-6}$)alkylCN (en particulier $CH_3CN$) et des ligands pyridine, éventuellement substitués, sur un ou plusieurs atomes de carbone des cycles pyridine, par un radical ($C_{1-6}$)alkyle ou un atome d'halogène, tel que défini ci-dessus.

Parmi d'autres ligands, on peut notamment citer les amines primaires ($C_{1-6}$)alkyle, tel que méthylamine ou éthylamine.

Les ligands donneurs de deux électrons par un atome de phosphore incluent les ligands de type phosphine. Avantageusement, ils sont de formule $P(Ph)_{3-x}(alkyle)_x$, avec x représentant 0, 1 ou 2 (de préférence x représente 2) (Ph représentant le groupe phényl). Parmi ces ligands, on peut notamment citer $P(Ph)(CH_3)_2$.

Selon un mode particulier, dans le cas de la formule (II), au moins deux des groupements $L_1$, $L_2$, $L_3$ et $L_4$, pris deux à deux, peuvent être reliés par au moins une liaison covalente. Dans ce cadre, on peut notamment citer les motifs bipyridine ou phénanthroline, éventuellement substitués, notamment par au moins un radical alkyle tel que défini ci-dessus. Dans le cas des ligands donneurs de deux électrons par un atome de phosphore, on peut citer avantageusement les ligands bidentés de formule $PR'_2$(alkylidène)$PR'_2$, avec R' représentant un groupe alkyle ou aryle (de préférence phényl) et le groupement alkylidène incluant des groupements de type $C_nH_{2n}$, ou $(CR^1R^2)_n$, avec n = 1 à 6 (de préférence 2 ou 3) et R1 et R2, identiques ou différents, représentant un groupe alkyle ou aryle tel que défini ci-dessus, le groupe alkylidène correspondant au lien covalent reliant au moins deux des groupements $L_1$, $L_2$, $L_3$ et $L_4$. Dans ce cadre, on peut notamment citer le ligand bidenté 1,2-bisdiphénylephosphinoéthane.

Ainsi, de manière préférentielle, les composés de l'invention présentent au moins un groupement $L_1$, $L_2$, $L_3$ et $L_4$, représentant un ligand donneur de deux électrons par un atome d'azote ou de phosphore, en particulier un groupement pyridine, phophine (e.g, de formule $P(Ph)_{3-x}(alkyle)_x$, telle définie ci-dessus), bipyridine ou phénanthroline, lesdits groupements étant éventuellement substitués.

Selon un autre mode particulier de l'invention, dans le cas de la formule (II), au moins deux des groupements $L_1$, $L_2$, $L_3$ et $L_4$, représentent des ligands nitriles, comme par exemple des ligands de formule ($C_{1-6}$)alkylCN (en particulier $CH_3CN$).

Selon un autre mode particulier de l'invention, dans le cas de la formule (II), deux des groupements $L_1$, $L_2$, $L_3$ et $L_4$, représentent des ligands nitriles, comme par exemple des ligands de formule ($C_{1-6}$)alkylCN (en particulier $CH_3CN$), et les deux autres ligands sont reliés par au moins une liaison covalente, avantageusement tels que ceux décrits ci-dessus.

$Y^-$ dans les composés selon l'invention est un contre-ion et est seulement présent dans le composé lorsque le complexe du ruthénium porte une charge positive. $Y^-$ est de préférence un anion peu nucléophile, tel que par exemple

$BF_4^-$, $PF_6^-$, $CF_3SO_3^-$, $CF_3CO_2^-$, $CH_3CO_2^-$ et $NO_3^-$, en particulier $PF_6^-$.

Selon un mode particulier de l'invention, m est égal à 1.

La ligne courbe représente avec les atomes de carbone, d'azote et de ruthénium représentés sur les formules (I) et (II), le métallocycle. Ce métallocycle est généralement constitué de 5 à 8 atomes (incluant les atomes de carbone, d'azote et de ruthénium représentés dans les formules (I) et (II)). Typiquement, les atomes du métallocycle (autres que ceux représentés dans les formules (I) et (II)) sont choisis parmi les atomes de carbone, d'azote, d'oxygène ou de soufre. Chacun de ces atomes peut indépendamment du métallocycle former des structures linéaires ou cycliques, saturées ou non, pour lesquelles il n'existe pas de limitations particulières.

Ainsi, parmi les unités structurales incluant un métallocycle à 5 ou 6 atomes (incluant les atomes Ru, C et N représentés dans les formules (I) et (II)), on peut notamment citer :

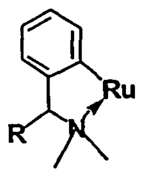

R = H, Me

(1)

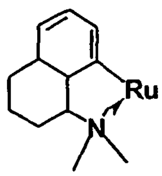

(2)

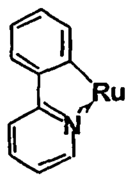

(3)

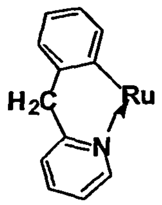

(4)

Parmi d'autres structures incluant un métallocycle à 6 ou 7 atomes, on peut notamment citer :

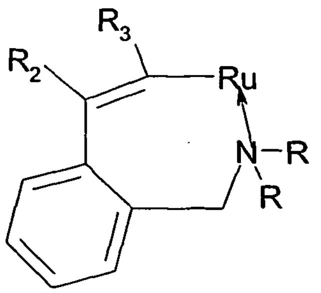

(5)

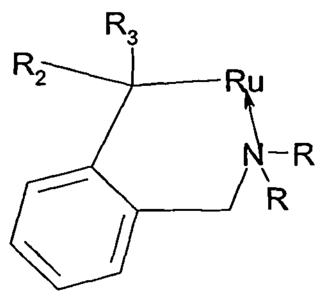

(6)

avec R, identique ou différent, représentant H ou un radical alkyle, de préférence méthyle, et $R_2$ et $R_3$, identiques ou différents, représentant un atome d'hydrogène, un atome d'halogène, un groupe alkyle tel que défini ci-dessus, un radical alkoxy (-O-alkyl), thiol, thioether (-S-alkyl), hydroxyl, nitro, cyano et ester ($-CO_2$-alkyl).

Dans le métallocycle (5), de préférence $R_2$ et $R_3$ représentent tous deux $CO_2Me$ et/ou les deux R représentent un radical méthyle.

Dans le métallocycle (6), de préférence $R_2$ représente H et $R_3$ un radical méthyle (Me) et/ou les deux R représentent un radical méthyle.

La présente invention concerne également les isomères optiques et géométriques, pris individuellement ou en mélange (notamment les racémates), des composés complexes du ruthénium (II).

Avantageusement, l'atome d'azote du métallocycle et représenté dans les formules (I) et (II) n'est pas un atome d'azote inclus dans une structure de type benzodiazépine, en particulier les composés décrits dans Organometallics, vol. 21, 2002, pp 5437-5438.

Selon un mode particulier de l'invention, les composés selon l'invention ne sont pas les composés suivants (décrits dans Organometallics, vol. 21, 2002, pp 1184-1189).

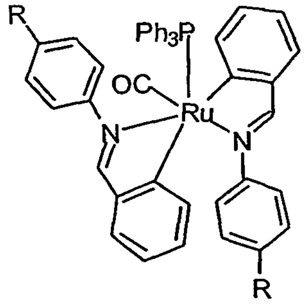

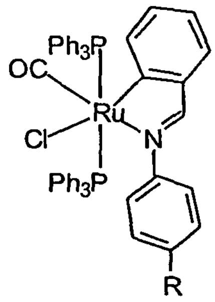

R = $NMe_2$, Me, I, $NO_2$

R = $NMe_2$, Me

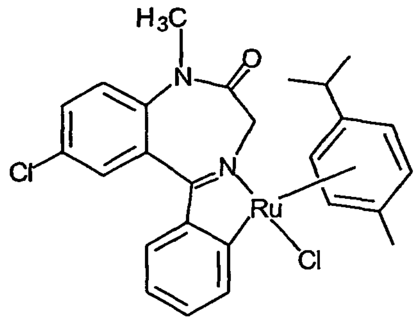

Parmi les composés de formule (I) ou (II), on peut citer notamment les composés représentés à la figure 8 (composés numérotés de 3-6 et 8-29). En particulier, les composés numérotés 9, 11, 12, 14-29 et, plus particulièrement, les composés 9, 11 et 12.

Ainsi, l'invention a également pour objet les composés 9, 11, 12 et 14-29. De préférence, les composés 9, 11, 12 et 28. Ces composés peuvent être utilisés à titre de médicaments et en particulier pour traiter des maladies liées à une hyperprolifération cellulaire, en particulier des cancers, tel que décrit dans la présente invention.

Il existe différentes voies de synthèse d'obtention des composés selon l'invention. Le procédé de préparation le plus avantageux est celui qui fait appel à la réaction dite de cyclométallation. Cette réaction met en jeu des propriétés chimiques particulières de la part des métaux à partir desquels cette réaction est observée. Certains métaux de transition sont en effet capables d'activer dans des conditions douces une liaison C-H grâce à laquelle ils substituent directement l'atome d'hydrogène pour former le composé cyclométallé. Le palladium est certainement le métal le plus couramment utilisé pour ce type de réaction. Plusieurs articles de revues (A. D. Ryabov, Chem. Rev. 1990, 90, 403-424) ont été consacrés à cette réaction et aux propriétés particulières des composés qui en résultent. Pour ce qui concerne les composés du ruthénium, une méthode de préparation de ces composés a été publiée dans Organometallics 1999, 18, 2390-2394. D'autres voies de synthèse sont également possibles notamment lorsque la réaction d'activation C-H directe par le métal de transition ne peut pas être mise en oeuvre. Dans ces cas-là, le coordinat organique azoté est métallé au niveau du carbone par le mercure(II) et le composé organomercuré ainsi obtenu peut être transmétallé sur le composé du ruthénium (voir J. Organometal. Chem. 1995, 494, 187-193, pour les composés du mercure, et Inorg. Chim. Acta 1996, 249, 63-67, pour la réaction de transmétallation).

Ainsi, les composés de la présente invention peuvent être obtenus à partir des familles de composés A et B décrites ci-dessous

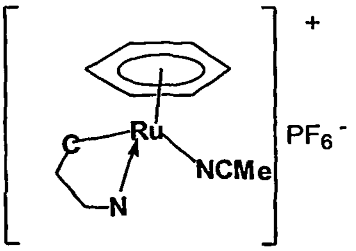

A

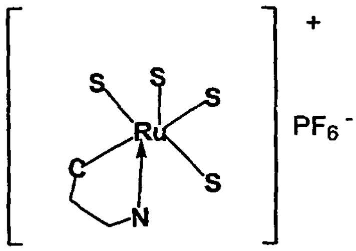

B

Dans ces familles de composés, l'unité "métallocyclique" représente en particulier et schématiquement les unités (1) à (6) décrites précédemment.

Les voies de synthèse pour obtenir ces deux familles de composés (A) ou (B) (incluses respectivement aux formules (I) et (II), avec S représentant un groupement acétonitrile $NCCH_3$) sont, soit la voie directe dite de cyclométallation par activation de la liaison C-H en ortho de l'aryle, soit la voie de transmétallation par un composé organomercuré. Le schéma général de synthèse des composés A et B est résumé sur le diagramme suivant :

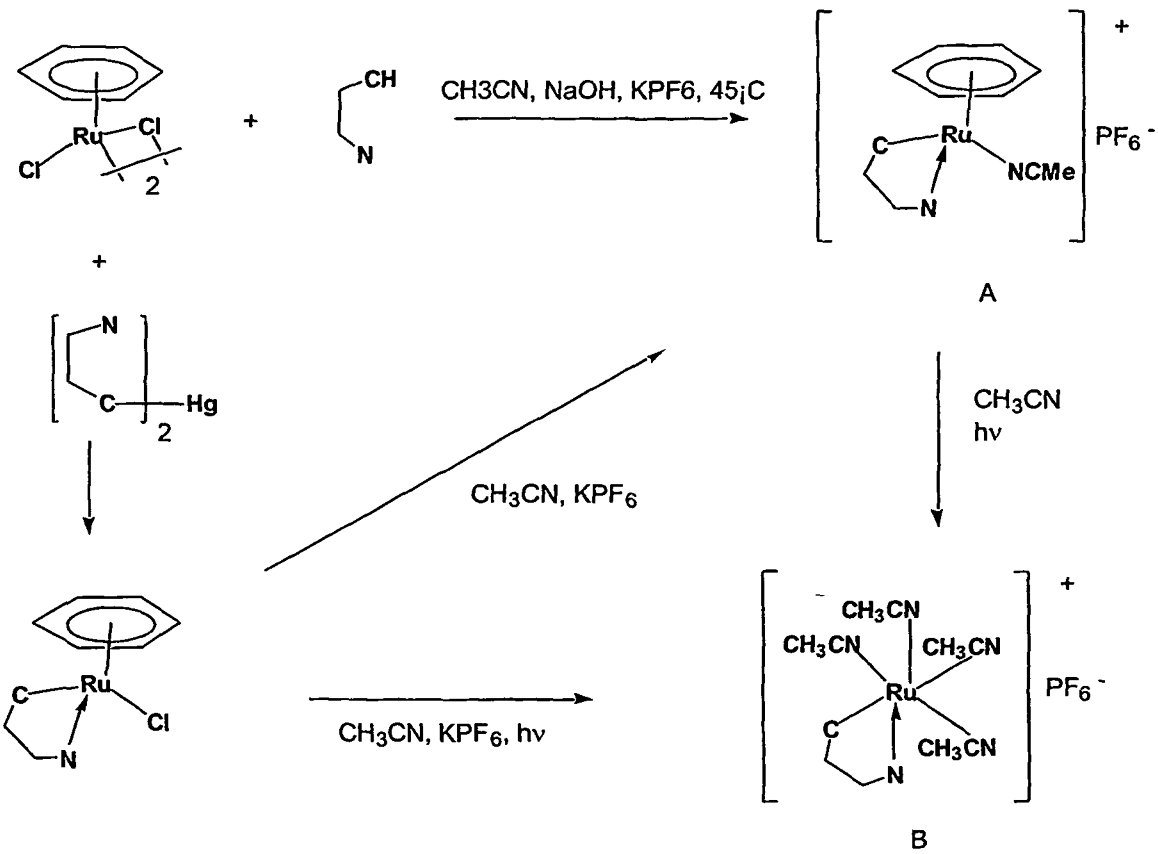


Les composés issus des familles A et B avec les différents coordinats organiques cyclométallés peuvent être modifiés en substituant un ou deux ligands acétonitriles par respectivement un ligand monodenté comme une phosphine $P(Ph)_{3-x}(alkyle)_x$, telle que définie ci-dessus, ce qui a conduit aux composés de type C et D, ou par un ligand bidenté comme une bipyridine ou la phénanthroline ou encore le ligand bidenté contenant du phosphore, comme le 1,2-bisdiphénylephosphinoéthane, ce qui a conduit aux composés de type E.

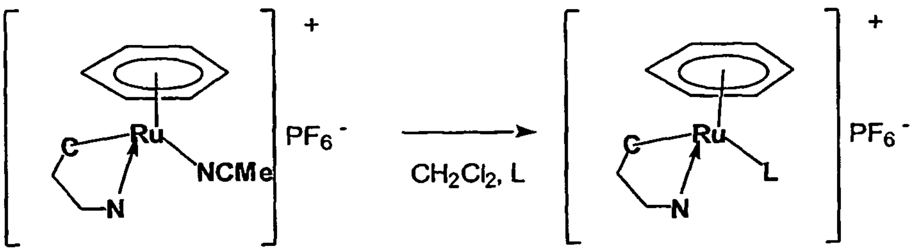


A C

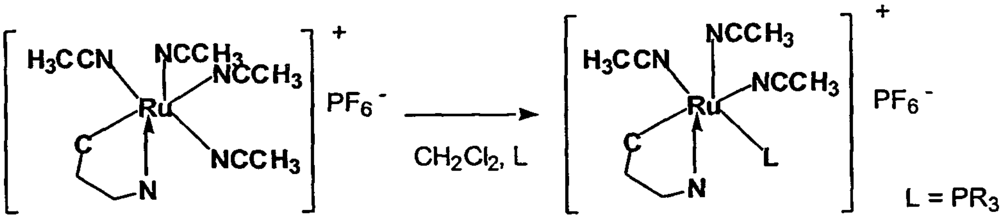


B D

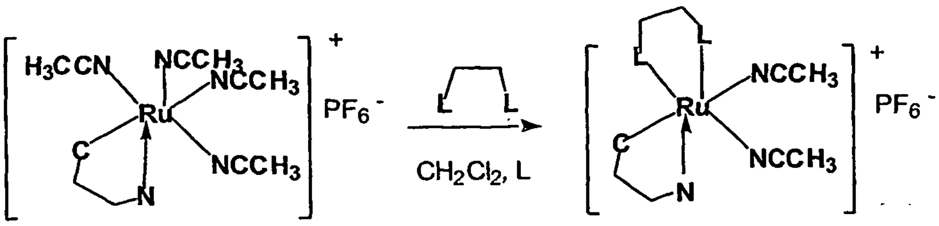


B E

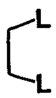 = bipyridine, ph˘nanthroline

Il existe de nombreux autres composés qui contiennent la même unité structurale métallocyclique. Par exemple, il est en effet possible de transformer les composés de type A par réaction avec un acétylénique ($R_2CCR_3$) ou un alcène ($R_2CH=CH_{2,}$ avec $R_2$ et $R_3$, tels que définis précédemment. Les composés ainsi formés ont une structure qui rappelle celle de A mais qui présente un groupe métallocyclique contenant un plus grand nombre d'atomes, 7 et 6 respectivement.

Les composés modifiés sont notamment les suivants :

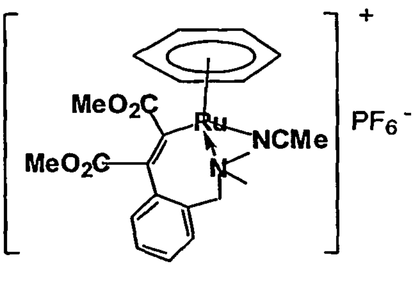

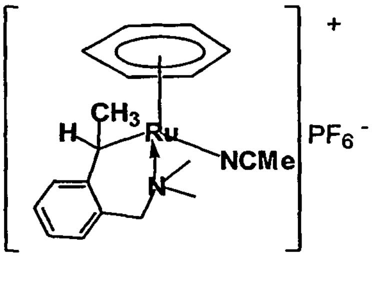

La synthèse de ces deux composés est décrite dans Bull. Soc. Chim. Fr. 1997, 134, 947-954 et dans Organometallics 2003, 22, 347-354. Les mêmes réactions que celles conduisant aux composés C, D et E peuvent être appliquées à ces espèces et ainsi élargir le nombre de composés présentant des propriétés anticancéreuses.

Comme spécifié ci-dessus, les compositions selon l'invention sont particulièrement avantageuses pour traiter des maladies liées à une hyperprolifération cellulaire, en particulier des cancers. Les cancers incluent ceux à tumeurs solides ou liquides. Les cancers correspondent notamment à des glioblastomes, des leucémies (promyélocytaires), les cancers de la prostate, des ovaires, des poumons, des seins, des voies digestives, en particulier du foie, du pancréas, de la tête et du cou, du colon, de la vessie, les lymphomes non-Hodgkiniens et les mélanomes.

La présente invention a également pour objet l'utilisation d'au moins un composé de formule (I) ou (II), tel que défini ci-dessus, dans le cadre de la préparation d'une composition pharmaceutique destinée à traiter des maladies liées à une hyperprolifération cellulaire, en particulier des cancers.

Les composés selon l'invention présentent un effet antiprolifératif vis-à-vis des cellules tumorales. Ils sont particulièrement utiles pour traiter des cancers par accumulation des cellules tumorales en phase G0/G1 et éventuellement par induction d'apoptose dans des cellules tumorales.

En effet, sans vouloir se lier à une quelconque théorie de l'invention, les composés selon l'invention semblent notamment capables d'accumuler les cellules tumorales en phase G0/G1, et donc en bloquant leur cycle cellulaire, mais également semblent capables de générer leur apoptose rapidement, en particulier lorsque leur concentration est augmentée, signe d'une toxicité dose-dépendante.

En outre, les composés selon l'invention sont particulièrement avantageux pour traiter des tumeurs résistantes au cisplatine ou à d'autres drogues anticancéreuses.

Les composés ou compositions selon l'invention peuvent être administrés de différentes manières et sous différentes formes. Ainsi, ils peuvent être administrés de manière systémique, par voie orale, par inhalation ou par injection, comme par exemple par voie intraveineuse, intra-musculaire, sous-cutanée, trans-dermique, intra-artérielle, etc., les voies intraveineuse, intra-musculaire, sous-cutanée, orale et par inhalation étant préférées. Pour les injections, les composés sont généralement conditionnés sous forme de suspensions liquides, qui peuvent être injectées au moyen de seringues ou de perfusions, par exemple. A cet égard, les composés sont généralement dissous dans des solutions salines, physiologiques, isotoniques, tamponnées, etc., compatibles avec un usage pharmaceutique et connues de l'homme du métier. Ainsi, les compositions peuvent contenir un ou plusieurs agents ou véhicules choisis parmi les dispersants, solubilisants, stabilisants, conservateurs, etc. Des agents ou véhicules utilisables dans des formulations liquides et/ou injectables sont notamment la méthylcellulose, l'hydroxyméthylcellulose, la carboxyméthylcellulose, le polysorbate 80, le mannitol, la gélatine, le lactose, des huiles végétales, l'acacia, etc.

Les composés peuvent également être administrés sous forme de gels, huiles, comprimés, suppositoires, poudres, gélules, capsules, aérosols, etc., éventuellement au moyen de formes galéniques ou de dispositifs assurant une libération prolongée et/ou retardée. Pour ce type de formulation, on utilise avantageusement un agent tel que la cellulose, des carbonates ou des amidons.

Il est entendu que le débit et/ou la dose injectée peuvent être adaptés par l'homme du métier en fonction du patient, de la pathologie concernée, du mode d'administration, etc. Typiquement, les composés sont administrés à des doses pouvant varier entre 0.1 $\mu$g et 100 mg/kg de poids corporel, plus généralement de 0,01 à 10 mg/kg, typiquement entre 0,1 et 10 mg/kg. En outre, des injections répétées peuvent être réalisées, le cas échéant. D'autre part, pour des traitements chroniques, des systèmes retard ou prolongés peuvent être avantageux.

L'invention concerne également une méthode de traitement d'une pathologie liée à une hyperprolifération cellulaire, en particulier un cancer, par l'administration à un sujet atteint d'une telle pathologie d'une quantité efficace de l'un des composés selon l'invention.

Dans le contexte de l'invention, le terme « traitement » désigne le traitement préventif, curatif, palliatif, ainsi que la prise en charge des patients (réduction de la souffrance, amélioration de la durée de vie, ralentissement de la progression de la maladie, réduction de la croissance tumorale, etc.). Le traitement peut en outre être réalisé en combinaison avec d'autres agents ou traitements chimiques ou physiques (chimiothérapie, radiothérapie, thérapie génique, etc.). Les traitements et médicaments de l'invention sont tout particulièrement destinés aux humains.

Ainsi, les composés selon l'invention peuvent être utilisés de manière avantageuse en combinaison avec un traitement anti-cancéreux mettant en oeuvre des rayonnements, tel que la radiothérapie et la curiethérapie. Les rayonnements mis en oeuvre sont en particulier les rayons X, les rayons gamma, des particules ionisantes tels que les électrons, les neutrons ou les ions carbone.

Selon un autre aspect de l'invention, les composés selon l'invention peuvent être utilisés avec d'autres agents chimiques ou traitements thérapeutiques anti-cancéreux, tel que les agents chimiques thérapeutiques suivants : le cisplatine, le carboplatine, le NCS (Néocarzinostatin), le taxotère ou le taxol, avantageusement le NCS ou le taxol. Les composés selon l'invention sont de préférence conditionnés et administrés de façon combinée, séparée ou séquentielle par rapport à d'autres agents ou traitements thérapeutiques.

Elle concerne aussi une méthode pour inhiber *in vivo*, *in vitro* ou *ex vivo* la prolifération de cellules tumorales comprenant la mise en contact desdites cellules tumorales avec l'un des produits selon l'invention. Les cellules tumorales

peuvent notamment provenir des pathologies spécifiées ci-dessus.

## LEGENDE DES FIGURES

Dans les figures et les exemples qui suivent, les termes « CDR » ou de manière équivalente « RDC » signifient « composé dérivé du ruthénium ».

***FIGURE 1 :*** Les cellules A172 ont été cultivées en plaques de 96 puits en milieu DMEM avec 10% de sérum ce veau. À 30% de confluence, les cellules ont été traitées pendant 48 h avec le cisplatine ou les divers composés dérivés du ruthénium aux concentrations indiquées (1, 5, 15, 50 μM). La quantité de cellules présentent dans les puits a été évaluée par un test MTT (MTT, Sigma) dont les produits de la réaction sont quantifiés avec un lecteur de plaque Elisa (Metertech, USA) (490-650 nm). Les résultats obtenus ont été rapportés aux valeurs de la condition contrôle (100 % de viabilité). Les graphiques sont une moyenne de 8 points avec les écarts types sur une expérience représentative sur 4 effectuées. La ligne noire épaisse indique l'IC50 pour chaque graphique.
***TABLEAU 2 :*** résumé des résultats obtenus sur diverses lignées cellulaires et des cultures primaires de glies. Les expériences ont été menées dans les mêmes conditions que celles décrites pour les cellules A172. Chaque IC50 est une valeur indicative obtenue sur 4 expériences indépendantes.

***FIGURE 2 :*** A- Les cellules A172 ont été cultivées sur lamelles de verre. À 50 % de confluence, les cellules ont été traitées pendant 24 h avec par le cisplatine, le CDR2 (25 μM) ou le CDR6 (15 μM). Les cellules ont ensuite été fixées au paraformaldéhyde 4%, perméabilitées au NP40 0,1 % et marquées avec le colorant Hoechst (en bleu) et l'anticorps anti-p20 (fragment actif de la caspase 3, en rouge). Les cellules ont ensuite été observées au microscope à fluorescence. B-Représentation graphique du nombre de cellules avec un noyau apoptotique, fragmenté ou condensé. Les barres sont une moyenne de deux lamelles d'une expérience représentative sur 3 réalisées. C- En parallèle, des extraits protéiques ont été préparés à partir de cellules traitées aux diverses drogues (cisplatine 15 μM ; CDR2 25 μM ; CDR6 15 μM ; CDR9 15 μM) et après dénaturation les extraits ont été déposés sur un gel SDS-Page 10 %. Après migration, les protéines ont été transférées sur nitrocellulose (Biorad, 0,2μm) et le fragment p20 de la caspase 3 a été détecté par Western blot avec un anticorps anti-p20 (1/1000, R&D System).

***FIGURE 3 :*** Détection de p53 et p73 dans les cellules A172 traitées aux CDR. Les cellules A172 ont été traitées aux concentrations indiquées (2, 5 ou 10 μM) avec les divers composés et pour les durées indiquées (24 h ou 6 h). Les protéines ont été extraites avec un tampon de lyse et ont été séparées sur gel SDS-Page 10%. Après transfert sur nitrocellulose, les protéines p53 et p73 ont été détectées par Western blot à l'aide d'anticorps anti-p53 (pAb1801) et anti-p73 (1/200, Ab2, Oncogene).

***FIGURE 4 :*** Détection de p21 et Bax dans les cellules A172 traitées aux CDR. Les cellules A172 ont été traitées avec les divers composés (10 μM) pendant 24 h. Les protéines ont été extraites avec un tampon de lyse et ont été séparées sur gel SDS-Page 10%. Après transfert sur nitrocellulose, les protéines p21 et Bax ont été détectées par Western blot à l'aide d'anticorps anti-p21 (1/200, Oncogene) et anti-Bax (1/2000, Santacruz).

***FIGURE 5 :*** Détection de p53, p21 et de la phosphorylation de l'histone H3 dans les cellules HCT116 traitées aux CDR. Les cellules HCT116 ont été traitées avec les divers composés (10 μM) et pour les durées indiquées (24 h ou 6 h). Les protéines ont été extraites avec un tampon de lyse et ont été séparées sur gel SDS-Page 10%. Après transfert sur nitrocellulose, les protéines p53, p21 et histone H3 ont été détectées par Western blot à l'aide d'anticorps anti-p53 (pAb1801), anti-p21 (1/200, Oncogene) et anti-phospho sérine 10 de l'histone H3 (1/2000, Santacruz).

***FIGURE 6 :*** Les cellules 2008/CMV et 2008/ATP7B (un don du Dr. Howell) ont été cultivées en plaques de 96 puits en milieu DMEM avec 10% de sérum de veau. À 30% de confluence, les cellules ont été traitées pendant 48 h avec le cisplatine ou les divers composés dérivés du ruthénium aux concentrations indiquées (1, 5, 15, 50 μM). La quantité de cellules présentent dans les puits a été évaluée par un test MTT (MTT, Sigma). Les résultats obtenus ont été rapportés aux valeurs de la condition contrôle (100 % de viabilité). Les graphiques sont une moyenne de 8 points avec les écarts types sur une expérience représentative sur 3 effectuées. La ligne noire épaisse indique l'IC50 pour chaque graphique.

***FIGURE 7 :*** Les cellules A172 ont été cultivées en plaques de 96 puits en milieu DMEM avec 10% de sérum ce veau. À 30% de confluence, les cellules ont été traitées pendant 48 h avec le cisplatine, le CDR6, le NCS (Néocarzinostatin), le Taxol ou une combinaison de ces drogues aux concentrations indiquées. La quantité de cellules présentent dans les puits a été évaluée par un test MTT (MTT, Sigma) dont les produits de la réaction sont quantifiés

avec un lecteur de plaque Elisa (Metertech, USA) (490-650 nm). Les résultats obtenus ont été rapportés aux valeurs de la condition contrôle (100 % de viabilité). Les graphiques sont une moyenne de 8 points avec les écarts types sur une expérience représentative sur 4 effectuées. La ligne noire épaisse indique l'IC50 pour chaque graphique.

***FIGURE 8 :*** Exemples de composés de formule (I) ou (II), à l'exception des composés numérotés 1 et 2 qui sont présentés à titre de références.

***FIGURE 9 :*** Effet d'une irradiation (4 Gy), d'un traitement par le RDC-11 (RDC-11/NI) et de l'association « irradiation + RDC-11 » (RDC-11/4 Gy) sur la prolifération des cellules RDM4 en culture.

***FIGURE 10 :*** Pourcentage d'apoptose sur des cellules RDM4 induite par le RDC-11 (RDC-11), par l'irradiation par des neutrons rapides (4 Gy), ou par la combinaison des deux traitements (4 Gy + RDC-11), trois jours après le début du traitement. L'analyse a été effectuée par cytométrie en flux, après marquage des cellules par l'iodure de propidium.

***FIGURE 11 :*** Pourcentage de viabilité de cellules HCT-116 en présence de RDC-11, RDC-24 et RDC-23 ou de Cisplatine à différentes concentrations.

***FIGURE 12 :*** Volume moyen ($mm^3$) de tumeurs apparentes de cellules U-87 (glioblastome humain) greffées sur souris athymiques SWISS nu/nu en fonction du temps (jours) après le début du traitement par le RDC-11 ou le D-PBS (contrôle).

***FIGURE 13 :*** Evolution des poids moyens de souris SWISS greffées avec des cellules U-87 (glioblastome humain) en fonction du temps (jours) après le début du traitement par le RDC-11 ou par le D-PBS (contrôle).

***FIGURE 14 :*** Poids des tumeurs (après dissection) de souris SWISS greffées avec des cellules U-87 (glioblastome humain) après un traitement par le RDC-11 ou par le D-PBS (contrôle).

D'autres aspects et avantages de la présente demande apparaîtront à la lecture des exemples qui suivent, qui doivent être considérés comme illustratifs et non limitatifs.

## EXEMPLES

### *1- PROCEDES DE SYNTHESE DE COMPOSES SELON L'INVENTION :*

Les composés cyclométallés du ruthénium(II) sont sensibles à l'oxygène de l'air et aux acides. Tous les solvants doivent par conséquent être parfaitement séchés et désoxygénés avant leur utilisation. Les manipulations se font dans une atmosphère controlée (azote ou argon) en utilisant la technique des tubes de schlenks.

Synthèse du composé **5** (d'après Organometallics, 1999, 18, 2390):

On ajoute la N,N dimethylaminomethylbenzene (0.120 mL, 0. 8 mmol), NaOH (0.031 g, 0.8 mmol) et KPF6 (0.292 g, 1.6 mmol) à une suspension de $[(\eta^6\text{-}C_6H_6)RuCl_2]_2$ (0.2 g, 0.4 mmol) dans l'acétonitrile (5mL) à 20 °C pendant 3 jours. La solution jaune ainsi obtenue est alors filtrée (chromatographiée) sur de l'alumine standardisée (12x3cm) avec l'acétonitrile comme éluant. On recueille la fraction jaune qui est sèchée sous vide. Le résidu obtenu est redissout dans un minimum d'acétonitrile (2mL). L'ajout d'éther éthylique à cette solution provoque la cristallisation d'un produit jaune vif qui est le composé attendu (rendement 0.32 g, 80%).

Synthèse du composé **6**

$[Ru\ (\eta^6\text{-}C_6H_6)\text{-}2\text{-}(CH_2NMe_2)\text{-}C_6H_4\text{-}(PMe_2Ph)]PF_6$ : on ajoute $PMe_2Ph$ (0,019mL, 0,162 mmol) à une solution jaune du complexe 5 (0,08g, 0,156 mmol) dans $CH_2Cl_2$ (10mL) et la solution résultante est agitée à température ambiante pendant 3 heures. Le solvant est évaporé sous vide et le résidu résultant est dissout dans un minimum de $CH_2Cl_2$ (1 mL). L'addition de n-hexane provoque la précipitation du complexe 6 (rendement : 0,09g, 95%). Analyse élémentaire. Calculé pour $C_{23}H_{23}NF_6P_2Ru$+1/2 $CH_2Cl_2$: C, 44.13; H, 4.69; N, 2.19. Trouvé: C, 44.30, H, 4.59; N, 2.16. RMN$^1$H ($CD_3CN$): 7.75 (dt, 1H, H6, $^3J$=7.5), 7.39 (tdd, 1H, *p*, $^3J$=7.5, $^4J$=1.7), 7.22 (td, 2H, *m*, $^3J$=8.0, $^4J$=2.0), 7.08 (tdd, 1 H, H4 ou H5, $^3J$=7.1), 7.00-6.88 (m, 3H, o et H4 or H5), 6.72 (d, 1H, H3, $^3J$=7.5), 5.74 (d, 6H, $C_6H_6$, $^3J_{HP}$=1.1), 2.85 and 2.43 ((AB, 2H, $^2J$=14.5), 2.77 (d, 3H, NMe, $^4J_{HP}$=1.1), 2.66 (s, 3H, NMe), 1.99 (d, 3H, PMe, $^2J$=9.3), 1.50 (d, 3H,

PMe, $^2J$=9.7).
RMN$^{31}$P ($CD_3CN$): 6.37 (s, 1P, $PMe_2$), -142.97 (sept, 1P, $PF_6$, $^1J$=711).

Synthèse du composé **8** (d'après Organometallics , 1999, 18, 2390):

On ajoute la 2-phenylpyridine (6,02 mmol), NaOH (0,48 g, 6,02 mmol) et $KPF_6$ (2,22 g, 12,04 mmol) à une suspension de [(eta$^6$-$C_6H_6$)$RuCl_2$]$_2$ (1,5 g, 3,01 mmol) dans l'acétonitrile (50mL) à 45 °C pendant 20h. Le solvant est alors évaporé sous vide et le résidu obtenu est chromatographié sur colonne d'alumine standardisé avec l'acétonitrile comme éluant. La bande jaune est recueillie et séchée sous vide. Ce résidu est dissout dans un mélange d'acétonitrile et de dichlorométhane (1:1); la diffusion lente d'ether ethylique dans cette solution permet d'obtenir le composé **8** sous la forme de cristaux jaunes vifs (rendement 68%).

Synthèse du composé **9**

[Ru ($C_6H_4$-2-$C_5H_4$N) ($PMe_2$Ph) ($NCMe)_3$]$PF_6$ **9:** on ajoute $PMe_2$Ph (0,031mL, 0,22 mmol) à une solution jaune du complexe **8** (0.124g, 0.22 mmol) dans $CH_3CN$ (5mL) et la solution résultante est agitée à température ambiante pendant 18 heures. La solution jaune-verte est filtrée sur alumine avec de l'acétonitrile comme éluant. La fraction jaune est récoltée et concentrée sous vide. La poudre obtenue est dissoute dans un minimum d'acétonitrile et d'éther éthylique. (1:1). L'addition de n-hexane provoque la précipitation du composé **9** sous la forme d'une poudre jaune. (rendement : 0.122g, 84%). Analyse élémentaire : Calculé pour $C_{25}H_{28}N_4F_6P_2$Ru: C, 45.39, H, 4.27, N, 8.47. trouvé: C, 45.35, H, 4.49, N, 8.33.
RMN$^1$H ($CD_3CN$): 8.39 (ddd, 1H, H12, $^3J$=5.8, $^4J$=1.6, $^5J$=0.8), 8.08 (dddd, 1H, H6, $^3J$=7.1, $^4J_{HP}$=4.7, $^4J$=1.3, $^5J$=0.5), 7.89 (d, 1 H, H9, $^3J$=8.1), 7.80 (d, 1 H, H3, $^3J$=7.8), 7.71-7.63 (m, 3H, H10 et *o*), 7.53-7.49 (m, 2H, m), 7.44 (t, 1H, *p*, $^3J$=7.4), 7.20 (td, $^1$H, H5, $^3J$=7.3, $^4J$=1.4), 7.06 (td, 1H, H4, $^3J$=7.5, $^4J$=1.4), 6.92 (ddd, 1H, H11, $^3J$=7.3, $^3J$=5.8, $^4J$=1.5), 2.33 (d, 3H, NCMe, $^5J_{HP}$=1.7), 1.97 (d, 3H, NCMe, $^5J_{HP}$=1.8), 1.86 (d, 6H, $PMe_2$, $^2J_{HP}$=5.8).
RMN $^{13}C\{^1H\}$ ($CD_3CN$): 185.5 (C1), 170.2 (C8), 156.6 (C12), 147.1 (C2), 138.0 (C6), 137.5 (C10), 131.0 (Co), 129.7 (Cm), 129.3 et 129.2 (C5 and Cp), 124.4 (C4 et *Cipso),* 123.6 (NCMe), 122.9 (C4), 122.3 (C11), 119.4 (C9), 13.55 and 13.41 ($PMe_2$), 4.31 et 3.99 (NCMe).
RMN$^{31}$P($CD_3CN$): -7.08 (s, 1P, $PMe_2$), -144.40 (sept, 1P, $PF_6$, $^1J$=704.6).

Synthèse du composé **11**

[Ru($C_6H_4$-2-$C_5H_4$N)(phen)($NCMe)_2$]$PF_6$ **11:** on ajoute la phénanthroline (0,032g, 0,178 mmol) au composé **8** (0,1g, 0,178 mmol) dissout dans $CH_2Cl_2$ (13 mL). La solution est agitée à temp. ambiante pendant 2 jours, l'évolution de la réaction étant suivie par RMN du proton. Le solvant est ensuite évaporé sous vide et le résidu est dissout dans le minimum d'acétonitrile/$CH_2Cl_2$ (1:1) et de n-hexane (20 mL) et cette solution est laissée au repos pendant 3 jours. Le produit **11** est obtenu sous forme de cristaux bruns foncés (rendement : 0,099g, 84%). Analyse élémentaire : Calculé. pour $C_{27}H_{22}N_5F_6$PRu: C, 48.95, H, 3.35, N, 10.57. trouvé: C, 48.00, H, 3.58, N, 10.60.
RMN $^1$H ($CD_3CN$): 9.72 (dd, 1H, H14, $^3J$=$_{5.0}$, $^4J$=$_{1.5}$), 8.72 (dd, 1 H, H16, $^3J$=8.2, $^4J$=1.4), 8.30 (dd, 1H, H23, $^3J$=7.4, $^4J$=1.3), 8.22 (m, 1H, H18), 8.20-8.17 (m, 2H, H15 et H11), 8.15 (dd, 1H, H12, $^3J$=5.3, $^4J$=1.4), 8.03 (d, 1H, H19, $^3J$=8.9), 7.88 (dd, 1H, H9, $^3J$=7.8, $^4J$=1.2), 7.84 (d, 1 H, H6, $^3J$=8.1), 7.48 (ddd, 1H, H5, $^3J$=8.1, $^3J$=7.4, $^4J$=1.6), 7.37 (dd, 1 H, H10, $^3J$=8.1, $^3J$=5.4), 7.34 (ddd, 1H, H3, $^3J$=5.7, $^4J$=1.6, $^5J$=0.8), 7.30 (dd, 1H, H21, $^3J$=7.3, $^4J$=1.3), 7.11 (dd, 1H, H22, $^3J$=7.7, $^3J$=7.2), 6.58 (ddd, 1H, H4, $^3J$=7.3, $^3J$=5.8, $^4J$=1.5), 2.29 (s, 3H, NCMe), 2.07 (s, 3H, NCMe).
RMN $^{13}C\{^1H\}$ ($CD_3CN$): 192.6 (C1), 169.5 (C8), 156.1 (C15), 152.0 (C3), 151.5 (C14), 150.7 (C2), 147.4 et 146.8 ( C26 et C25), 139.0 (C23), 136.5 (C16), 136.4 (C5), 135 (C18), 131.3 et 131.1 (C20 et C17), 129.2 (C21), 128.4 (C12), 128.3 (C19), 126.8 (C11), 125.1 (C10), 124.7 (C9), 125.5 et 122.7 (NCMe), 121.9 (C4), 121.5 (C22), 118.8 (C6), 4.40 et 4.01 (NCMe).

Synthèse du composé **12 :**

[Ru($C_6H_4$-2-$C_5H_4$N)(4,4'-dimethyl-2,2'-bipyridine)($NCMe)_2$]$PF_6$ **12:** on ajoute la 4,4'-diMe-2,2'-bipyridine (0,032g, 0.181 mmol) au composé **8** (0,102g, 0.181 mmol) dissout dans $CH_2Cl_2$ (13 mL). La solution est agitée à température ambiante pendant 2 jours, l'évolution de la réaction étant suivie par RMN du proton. Le solvant est ensuite évaporé sous vide et le résidu est dissout dans le minimum d'acétonitrile/$Et_2O$ (1:1) et de n-hexane (30 mL) et cette solution est laissée au repos pendant 3 jours. Le produit **11** est obtenu sous forme de cristaux bruns foncés Anal. Calcd. pour $C_{27}H_{26}N_5F_6$PRu C, 48.65, H, 3.93, N, 10.51. trouvé: C, 48.83, H, 4.32, N, 10.30.
RMN$^1$H ($CD_3CN$): 9.18 (d, 1H, H14, $^3J$=5.5), 8.30 (s, 1 H, H16), 8.21 (ddd, 1 H, H6, $^3J$=7.4, $^4J$=1.3, $^5J$=0.5), 8.09 (s,

1H, H17), 7.85-7.81 (m, 2H, H9 et H12), 7.68-7.66 (m, 2H, H15 et H19), 7.52 (ddd, 1H, H10, $^3J$=8.2, $^3J$=7.4, $^4J$=1.6), 7.44 (ddd, 1H, H3, $^3J$=5.7, $^4J$=1.6, $^5J$=0.8), 7.24 (td, 1 H, H11, $^3J$=7.3, $^4J$=1.3), 7.05 (ddd, 1H, H5, $^3J$=7.7, $^3J$=7.2, $^4J$=1.3), 6.85 (dd, 1H, H18, $^3J$=5.9, $^4J$=1.8), 6.73 (ddd, 1H, H4, $^3J$=7.3, $^3J$=5.7, $^4J$=1.5), 2.64 et 2.34 (2s, 6H, $CH_3$), 2.21 et 2.19 (2s, 6H, NCMe).

Synthèse du composé **13**

[Ru-2-($CH_2NMe_2$)-$C_6H_4$-(bipy) $(NCMe)_2$]$PF_6$

On ajoute un équivalent de 2,2'-bipyridine à une solution de **5** (0,1 g) dans 15mL d'acétonitrile. Le mélange est agité pendant 24h. La solution pourpre obtenue est évaporée sous vide et le résidu est chromatographié sur une colonne d'alumine avec l'acétonitrile comme éluant. La bande pourpre foncée est recueillie et évaporée sous vide. Le composé **13** est obtenu sous la forme de cristaux pourpres foncés en faisant diffuser de l'éther éthylique dans une solution de **13** dissout dans le minimum de dichlorométhane/acétonitrile (1:1) (rendement: 53%). Analyse élémentaire : Calculé. pour $C_{29}H_{38}N_5F_6PRu$: C, 49.57, H, 5.45, N, 9.97. trouvé: C, 49.21, H, 5.60, N, 9.62.
RMN $^1H$ ($CD_3CN$): 9.52 (dd, 1H, $^3J$ 6.0, $^4J$ 1.6, H2"), 8.69 (dd, 1H, $^3J$ 5.5, $^4J$ 1.1, H2'), 8.65 (d, 1H, $^3J$ 8.2, H"), 8.53 (d, 1 H, $^3J$ 7.7, H5'), 8.17 (td, 1H, $^3J$ 7.7, $^4J$ 1.1, H4"), 7.92 (td, 1H, $^3J$ 8.2, $^4J$ 1.6, H4'), 7.86 - 7.80 (m, 2H, H3" + H5), 7.36 (ddd, 1H, $^3J$ 7.1, $^3J$ 6.0, $^4J$ 1.6, H3'), 7.08 (td, 1 H, $^3J$ 7.1, $^4J$ 1.1, H4), 7.00 (d, 1H, $^3J$ 7.2, H2), 6.85 (td, 1 H, $^3J$ 7.1, $^4J$ 1.1, H3), 3.88 (d, 1 H, $^2J$ 13.8, $CH_2$), 3.30 (d, 1H, $^2J$ 13.8, $CH_2$), 2.45 (s, 3H, NCMe), 2.18 (s, 3H, $NMe_2$), 2.07 (s, 3H, NCMe), 1.36 (s, 3H, $NMe_2$).
RMN $^{13}C$ ($CD_3CN$): 168.46, 159.49, 155.80, 153.17 (C2'), 150.65 (C2"), 148.03, 137.61 (C5), 135.96 (C4"), 134.96 (C4'), 126.52 (C3"), 125.43 (C3'), 125.28 (C4), 122.82 (C5'), 122.60 (C5"), 120.76 (C2), 120.27 (C3), 118.03, 73.02 ($CH_2$), 52.00 ($NMe_2$), 50.46 ($NMe_2$), 4.00 (NCMe), 3.16 (NCMe).

Synthèse des composés **14** à **25 :**

Les composés **14** à **25** ont été obtenus par une méthode analogue à celle utilisée pour la synthèse de **13.** Dans un premier temps les composés analogues au composé **5** ont été obtenus en utilisant à la place de la N,N dimethylaminomethylbenzene le ligand substitué correspondant. Dans un deuxième temps, ces composés ont été traités par la 2,2'-bipyridine comme dans l'exemple ci-dessus, les rendements sont analogues à ceux obtenus pour **13.**

Synthèse du composé **26**

On synthétise d'abord le composé [($\eta^6$-$C_6H_6$)Ru($C_6H_4$-2-$CHCH_3NMe_2$)(NCMe)]$PF_6$ par une méthode analogue à celle utilisée pour synthétiser le composé **5.** Dans ce protocole, on substitue la N,N diméthylaminométhylbenzène par la (R) (1,1-phényléthyldiméthylamine) (0.8 mmol) et on opère exactement comme pour la synthèse de **5,** les rendements étant les mêmes. On ajoute ensuite la 2,2'-bipyridine (0.030 g, 0.19 mmol) à une solution de [($\eta^6$-$C_6H_6$)Ru($C_6H_4$-2-$CHCH_3NMe_2$)(NCMe)]$PF_6$ dans 15 mL d'acétonitrile. La solution résultante pourpre est agitée pendant 12 h. Le solvant est alors évaporé sous vide et le composé **26** est purifié par le biais d'une chromatographie sur alumine, l'éluant étant le dichorométhane. On recueille la bande pourpre qui est évaporée sous vide. La diffusion lente de diéthyle éther dans une solution du résidu obtenu après cette dernière opération dans $CH_2Cl_2$ :MeCN (1 :1) permet d'obtenir **26** sous la forme de cristaux pourpres avec un rendement de 53% (0.064g)
$^1H$-NMR ($CD_3CN$) $\delta$: 9.33 (ddd, 1H, H6' $^3J$= 5.3, $^4J$= 1.5, $^5J$=0.7), 8.44 (d, 1 H, H3' $^3J$= 8.2), 8.31 (d, 1 H, H6" $^3J$= 7.9), 8.18 (ddd, 1H, H6 $^3J$= 5.6, $^4J$= 1.5, $^5J$=0.6), 8.08 (ddd, 1H, H4' $^3J$=9.15, $^3J$=7.6, $^4J$=1.5), 7.79 (m, 2H, H5" and H4), 7.73 (ddd, 1H, H5' $^3J$= 7.5, $^3J$= 5.2, $^4J$= 1.1), 7.17 (ddd, 1 H, H5 $^3J$= 7.3, $^4J$= 1.4), 7.07 (m, 1 H, H4"), 6.92 (m, 2H, H3 and H3"), 3.40 (q, 1H, CH $^3J$= 6.6), 2.41 (s, 3H, $CH_3CN$), 2.04 (s, 3H, $NCH_3$), 1.96 (s, 3H, $CH_3CN$), 1.49 (s, 3H, $NCH_3$), 1.18 (d, 3H, $CH_3$ $^3J$=6.7).
Anal. Calc. pour $C_{24}H_{28}N_5F_6PRu$: C, 45.57, H, 4.46, N, 11.07. Trouvé: C, 45.59, H, 4.51, N, 10.93.
Le composé **27** est obtenu de la même façon en substituant la (R) (1,1-phényléthyldiméthylamine) par la (S) (1,1-phenylethyldimethylamine).

Synthèse du composé **28**

On ajoute la 1,2-bis,diphenylphosphinoéthane (($P(C_6H_5)_2CH_2CH_2P(C_6H_5)_2$) (0.141g, 0.35mmol) à une solution du composé **8** (0.2g, 0.35 mmol) dans le méthanol (30 mL). Cette solution jaune est agitée et chauffée au reflux du méthanol pendant 19 h. Le méthanol est alors évaporé sous vide et le composé **28** est purifié par chromatographie sur alumine, l'éluant étant le $CH_2Cl_2$. La fraction jaune est recueillie, concentrée sous vide, l'addition de diéthyle éther provoquant la précipitation du produit attendu qui est ensuite lavé trois fois avec de l'éther éthylique (rendement 46%,

0.143g).
$^1$H-NM R ($CD_3CN$) δ: 8.94 (m, 1H, H6), 8.07 (d, 1H, H12 $^3$J= 8.2), 7.95-7.84 (m, 3H, H9, H3 and H10), 7.64-7.36 (m, 20H, $PPh_2$), 7.07-7.01 (m, 2H, H5 and H4), 6.88 (ddd, 1H, $^3$J=7.3, H11 $^3$J= 6.0, $^4$J= 1.3), 2.69 (m, 4H, $CH_2$), 1.50 (d, 6H, $^5J_{H-P}$= 1,1 Hz, $CH_3CN$).
$^{31}$P-NMR ($CD_3CN$) δ: 68.41 (s, $PPh_2$), 43.64 (s, $PPh_2$), -143.3 (sept, $PF_6$).

Synthèse du composé **29**

On ajoute la triphénylphosphine (0.186g, 0.71mmol) à une solution du composé **8** (0.2g, 0.35 mmol) dans le méthanol (30 mL). Cette solution jaune est agitée et chauffée au reflux du méthanol pendant 72 h. Le méthanol est alors évaporé sous vide et le composé **29** est purifié par chromatographie sur alumine, l'éluant étant le $CH_2Cl_2$. La fraction jaune est recueillie et séchée sous vide. Le solide résultant est dissout dans un mélange de $CH_2Cl_2$ :MeCN (1 :1) auquel l'ajout de diéthyl éther provoque la cristallisation de **29** (rendement 37%, 0.131g).
$^1$H-NMR ($CD_3CN$) δ: 7.95 (dd, 1H, H6 $^3$J= 5.9, $^4$J= 0.7), 7.52 (m, 1H, H12), 7.4-7.0 (m, 33H, o, m, p $PPh_3$, H10, H9 and H3), 6.75 (ddd, 1H, H5 or H4 $^3$J=8.2, $^3$J= 7.9), 6.67 (ddd, 1 H, H4 or H5 $^3$J= 8.1 , $^4$J= 1.5), 6.47 (ddd, 1H, H11 $^3$J= 8.1), 2.14 (s, 3H, $CH_3CN$), 1.96 (s, 3H, $CH_3CN$).
$^{31}$P-NMR ($CD_3CN$) δ: 35.3 (s, $PPh_3$), -143.4 (sept, $PF_6$).
Anal. Calc. pour $C_{51}H_{44}N_3F_6P_3Ru$ : C, 60.83, H, 4.40, N, 4.17. Trouvé : C, 61.15, H, 4.54, N, 4.46.

## *2. TEST DE PROLIFERATION DES COMPOSES SELON L'INVENTION SUR DES LIGNEES CELLULAIRES TUMORALES*

Cellules

Les produits sont testés sur différentes lignées cellulaires. Les cellules utilisées sont des RDM4 issues d'un lymphome T murin ainsi qu'une lignée MOLT-4, provenant d'une leucémie lymphoblastique aiguë. Ces lignées cancéreuses expriment toutes les deux la protéine p53, contrairement aux cellules HL-60 issues d'une leucémie promyélocytaire humaine où, à la suite d'une délétion de son gène, p53 est absente.
L'influence de la protéine p53 dans les effets induits par les produits a été déterminée en utilisant la lignée lymphoblastoide humaine TK6 (*p*53 sauvage) et son variant NH32 dont le gène *p*53 a été totalement inactivé par double recombinaison homologue (Chuang *et al*, 1999). Les cellules TK6 proviennent de l'American Tissue Culture Collection (ATGC, Manassas, VA, USA). Les cellules NH32 ont été fournies par H. L. Liber (Chuang *et al*, 1999).
Toutes ces cellules sont cultivées dans du RPMI 1640-Glutamax, supplémenté par 10% de sérum foetal de veau inactivé à la chaleur à 56°C pendant 30 minutes, 1mM de pyruvate de sodium, 1mM d'acides aminés non essentiels et 50μg/mL de gentamycine (Life Technologies, Cergy Pontoise, France). Les cultures sont maintenues dans un incubateur à 37°C avec une atmosphère saturée d'humidité, à 5% de $CO_2$.
La concentration et la viabilité des cellules sont déterminées par test d'exclusion au Bleu de Trypan (Sigma-Aldrich, France) et la densité cellulaire est maintenue à une concentration inférieure à $10^6$ cellules/mL.

Test de prolifération

Ce test utilise le réactif UptiBlue (Interchim, Montluçon, France), métabolisé par les cellules vivantes. Les cellules sont incubées en plaques de 96 puits à $10^4$ cellules/puit (200μL) avec le produit et cultivées pendant 72 heures. Puis 20μL d'UptiBlue sont ajoutés dans chaque puits. Après 3 heures d'incubation, la fluorescence des échantillons contenus dans les plaques est mesurée à 590nm (excitation à 560nm), à l'aide d'un lecteur de micro-plaques Fluorolite 1000 (Dynex technologies, Issy-Les-Moulineaux, France).

## *RESULTATS*

Les résultats sont rassemblés dans le tableau 1 suivant.

Tableau 1

| | IC50 (μM) |
|---|---|
| Composés du ruthénium | Lignée RDM4 |
| **1** | 40 |
| **2** | - |

(suite)

| | IC50 (μM) |
|---|---|
| Composés du ruthénium | Lignée RDM4 |
| **3** | - |
| **4** | 50 |
| **5** | 40 |
| **6** | - |
| **8** | >50 |
| **9** | 12 |
| **10** | >50 |
| **11** | 11 |
| **12** | 9 |
| **13** | 11 |
| **14** | 15 |
| Cisplatine | 0,3 |

Le composé 9 sur d'autres lignées cellulaires donne les IC50 suivantes : **9** (NH32) : 3 ; **9** (Molt-4) : 3,8 ; **9** (WTK1) : 3 ; **9** (U-937) : 3.
RDM4: lymphome, souris AKR
TK6 : lignée lymphoblastoide humaine
NH32 : variant de TK6, n'exprimant pas p53 ("p53 knock-out")
WTK1 : autre variant de TK6, exprimant un gène p53 muté
MOLT-4 : leucémie lymphoblastique T humaine
U-937 : leucémie promonocytaire humaine

*- Test de prolifération de la lignée RDM4 avec chaque composé*

La sélection des anti-cancéreux potentiels est faite, dans un premier temps, sur la lignée RDM4. L'effet des différents composés organo-ruthénés sur la viabilité cellulaire est d'abord déterminé par un test de prolifération en fonction de la concentration en produit. L'activité des composés dérivés du Ru est comparée à celle du cisplatine. L'exposition des cellules RDM4 à ces dérivés se traduit par une diminution dose-dépendante de leur prolifération. L'effet dépend donc du complexe organique qui entoure le noyau de Ru. La concentration d'inhibition de la croissance de 50% des cellules (IC50) est déterminée pour chacun des produits. De tous les composés testés, les composés **9** et **11** apparaissent comme étant les plus actifs avec une IC50 comprise entre 10 et 15μM. Les dérivés du ruthénium montrant une IC50 trop importante (>50μM) ont été éliminés de l'étude biologique

*- Mesure de l'apoptose par marquage à l'annexine- V sur des RDM4 traitées avec les complexes 9 et 11*

La mesure de la mort cellulaire est faite par un premier marquage spécifique de l'apoptose. L'externalisation des phospholipides anioniques, normalement situés sur le feuillet interne de la membrane plasmique, est un des marqueurs précoces de l'apoptose (Martin *et al*, 1996). L'annexine-V est une protéine qui se fixe spécifiquement sur les phosphatidyl-sérines en présence de calcium. Lorsque l'annexine-V est couplée à un fluorochrome, il permet de quantifier les cellules apoptotiques par cytométrie en flux.

Les cellules sont marquées après 24h, 48h et 72h de traitement avec les produits **9** et **11** à deux concentrations différentes: l'IC50 (15μM) et une concentration supérieure qui garantit une inhibition importante de la prolifération (45μM). Les cellules contrôles sont traitées avec l'équivalent en volume de solvant (éthanol). Le traitement avec 15μM de produit ne montre pas d'effet. Après 72h, le taux d'apoptose est toujours aussi faible que celui des cellules contrôles. En revanche, avec le traitement à 45μM, ce taux atteint déjà 70% à 24h et dépasse 99% après 48h, signe d'une induction d'apoptose importante.

*- Mesure de la fragmentation de l'ADN par marquage à l'iodure de propidium sur des RDM4 traitées avec les complexes **9** et **11***

Un second marquage spécifique de l'apoptose est la fragmentation de l'ADN entre les nucléosomes. L'ADN fragmenté se retrouve dans les corps apoptotiques en phase terminale de l'apoptose. Cet ADN est hypodiploïde et donc de quantité inférieure à celle présente dans les cellules normales. Il peut être quantifié par cytométrie en flux après une perméabilisation de la membrane des cellules et un marquage à l'IP. La quantité d'ADN dans une cellule normale est de 2n en phase G0/G1 et de 4n en phase G2. L'ADN sub-G0 de quantité inférieure à 2n présente donc une intensité de fluorescence plus faible.

Les cellules traitées avec 15μM de produit présentent une accumulation en phase G0/G1 pendant les deux premiers jours de l'expérience. Ce phénomène tend à s'estomper après 48h de traitement. En revanche lorsque les cellules sont traitées à 45μM, la formation des particules hypodiploïdes, visualisées par le contenu en ADN sub-G0, est inférieure à 10% à 24h et dépasse les 50% à 48h pour atteindre les 60% à 72h. La fragmentation de l'ADN montre que l'apoptose induite par les produits survient rapidement. La quantité de particules hypodiploïdes chez les cellules contrôles reste inférieure à 4% pendant cette même période.

Les deux complexes organo-ruthénés présentant un effet antiprolifératif sont capables d'accumuler les cellules RDM4 en phase G0/G1, mais également de générer leur apoptose rapidement à plus forte concentration signe d'une toxicité dose-dépendante.

## *3. APOPTOSE ET PROTEINE P53*

Le rôle de la protéine p53 est central dans la gestion de l'apoptose et l'induction du blocage cellulaire. En cas d'altération de l'ADN cellulaire, cette protéine est un facteur transcriptionnel qui régule l'expression d'autres protéines intervenant dans le blocage du cycle, la réparation de l'ADN et dans l'induction de l'apoptose (Alarcon-Vargas & Ronai, 2002).

*- Effets comparés du complexe 11 et du cisplatine sur la prolifération des lignées MOLT- 4 et HL- 60*

Les cellules MOLT-4 possèdent le gène p53 qui est supprimé par délétion chez les HL-60. Les résultats des tests de prolifération comparés montrent que le produit 11 et le cisplatine ont des incidences différentes sur l'activité proliférative de ces lignées. Le cisplatine diminue la prolifération des cellules MOLT-4 de façon plus importante, (IC50 < 0,5μM) que celle des HL-60 *p*53 déficientes (leucémie promyélocytaire humaine) (IC50 = 1μM). Cette sensibilité est inversée avec le complexe de Ru. L'inhibition de la croissance induite par le Ru est plus importante pour les HL-60 que pour les MOLT-4. Ces dernières, qui ont un gène p53 normal, sont moins sensibles au complexe du Ru. La différence de prolifération en présence de cisplatine pourrait être expliquée par un retard du déclenchement de l'apoptose chez les cellules HL-60 par rapport aux MOLT-4 avec l'activation d'une voie secondaire indépendante de la protéine p53 (Coelho *et al*, 2002). Dans le cas du traitement avec le produit organo-ruthéné **11,** cette variation de sensibilité peut être imputée au statut de p53 dans la cellule ou à la différence du type cellulaire.

*- Effets comparés du complexe **11** et du cisplatine sur la prolifération des lignées TK6 et NH32*

Le rôle de la protéine p53 sur la prolifération cellulaire en présence du complexe **11** et du cisplatine est également étudié sur d'autres lignées cellulaires : les cellules lymphoblastoïdes humaines TK6 *(p53* sauvage) et leurs variants NH32 (*p*53 -/-). Les courbes de prolifération des deux types cellulaires peuvent être superposées. La variation du statut de la protéine p53 ne semble pas avoir d'effet sur l'action anti-proliférative du complexe de Ru. Par contre le cisplatine provoque un effet plus important sur les cellules p53 +/+ que sur les cellules p53 déficientes. p53 joue donc un rôle dans le pouvoir inhibiteur du cisplatine. Cependant l'action anti-proliférative du cisplatine est moins importante que celle du complexe **11.**

*- Mesure de l'apoptose par marquage à l'annexine-V sur des cellules TK6 et NH32 traitées avec le complexe **11***

La toxicité du complexe organo-ruthéné sur les cellules lymphoblastoïdes TK6 et NH32 est mesurée avec l'annexine-V, marqueur spécifique de l'apoptose précoce. Ce marquage est effectué sur des cellules traitées avec le dérivé **11** à 1,5μM après 0h, 24h, 48h, 72h et 96h. Les résultats montrent que l'externalisation des phosphatidyl-sérines dépasse 50% à 48h et atteint 90% après 72h de traitement continu. Au-de là de 72h, l'apoptose atteint la limite maximum de 90% et forme un plateau. Chez les cellules contrôles, le taux d'apoptose reste inférieur à 10% tout au long de l'expérience. L'augmentation avec le temps de l'externalisation des phospholipides anioniques est similaire chez les deux lignées cellulaires, mais les cellules TK6 entrent en apoptose avec un retard d'une douzaine d'heures sur leurs

variants *p53* déficients. La lignée NH32 atteint la limite formée par le plateau de 90% plus tôt que les cellules TK6. La protéine p53 semble retarder l'activation des signes précoces de l'apoptose induite par les complexes du Ru.

***4. ASSOCIATION CHIMIO-RADIOTHERAPEUTIQUE***

- Les IC50 du cisplatine et du complexe organo-ruthéné **11** mesurées sur les RDM4 sont respectivement d'environ 0,7μM et 15μM. Des cellules de cette même lignée sont traitées avec des concentrations de 0,7μM de cisplatine et 15μM de complexe **11.** 24 heures après le début du traitement, les cellules sont irradiées à 4Gy avec des neutrons rapides. Les effets de l'association chimio-radiothérapeutique sont alors déterminés par simple comptage cellulaire en mesurant la concentration de cellules dans le milieu au cours du traitement à 24h, 72h et 168h après l'irradiation. Les échantillons non-irradiés, traités et non-traités, ont tous une prolifération exponentielle. Cette croissance est ralentie par l'ajout des complexes chimiques organo-métalliques. Le cisplatine à 0,7μM inhibe la prolifération plus efficacement que le dérivé ruthéné à 10μM. L'association chimio-radiothérapeutique augmente l'efficacité des composés chimiques et montre un effet supérieur à une simple addition des effets des radiations ionisantes et des complexes pris séparément. La courbe du dérivé ruthéné **11** seul se superpose à celle des cellules contrôles. L'effet antiprolifératif de ce complexe à cette concentration est négligeable. Mais l'addition de radiations ionisantes à ce produit entraîne un effet radical rendant la prolifération cellulaire quasiment nulle pendant la durée de l'expérience. Cette observation ne se retrouve pas de façon aussi importante avec le cisplatine qui, lorsqu'il est utilisé seul, a un effet cytostatique plus marqué que celui du complexe **11.**

- Evaluation des effets d'un traitement combiné « radiations ionisantes + RDC-11 » sur la prolifération et la survie de cellules murines RDM4. Ces cellules proviennent d'un lymphome de la souris AKR. Les différents tests ont été effectués *in vitro*, et cinq expériences indépendantes ont été réalisées.

Les radiations utilisées étaient des neutrons rapides provenant de la collision de protons de 65 MeV sur une cible de Béryllium (produits au cyclotron de Louvain la Neuve (LLN) en Belgique. Les résultats d'une seule de ces expériences, qui est représentative d'un ensemble de trois expériences faites avec ce type de rayonnement, sont présentés. Des résultats similaires ont été observés avec des rayons X (produits au centre Paul Strauss, Strasbourg), et des ions carbone (produits au GANIL, Caen).

Protocole expérimental

Les cellules RDM4 sont ajustées à 50.000 cellules/ml, dans du milieu de culture RPMI 1640 additionné de 10% de sérum de veau foetal. 4 Flacons de culture de 25 cm2 sont remplis de suspension cellulaire, à raison de 10 ml/flacon, soit 500.000 cellules/flacon.

Quatre flacons sont ainsi préparés, correspondant chacun à un groupe expérimental différent :

Groupe 1 : cellules non-irradiées, non traitées (Et/NI dans la figure 9)
Groupe 2 : cellules non-irradiées, traitées par le RDC-11 (RDC-11/NI dans la figure 9)
Groupe 3 : cellules irradiées, non traitées (Et/4 Gy dans la figure 9)
Groupe 4 : cellules irradiées, traitées par le RDC-11 (RDC-11/4 Gy dans la figure 9)

Le RDC-11, préparé à partir d'une solution éthanolique, est ajouté aux cellules (flacon 2 et 4) 6 heures avant l'irradiation. Un volume identique d'éthanol (Et, 66 μl) est ajouté aux flacons 1 et 3. La concentration finale de RDC-11 est de 10 μM, et le milieu n'est pas remplacé durant les 9 jours de l'expérience.

Les flacons 3 et 4 sont irradiés à 4 Gy, à température ambiante, puis remis en culture à 37°C.

Les jours suivant l'irradiation, des aliquotes de suspensions cellulaires sont régulièrement prélevées. Le nombre de cellules est déterminé à l'aide d'un Coulter Counter. D'autres cellules sont fixées dans de l'éthanol, puis marquées par l'iodure de propidium, afin de déterminer le pourcentage d'apoptose.

Résultats

Les comptages cellulaires font clairement apparaître l'action des radiations et du RDC-11 sur la prolifération des RDM4. Lorsque ces deux traitements sont combinés, la croissance cellulaire est fortement ralentie. Cet effet est particulièrement marqué à 9 jours post-irradiation (figure 9).

L'analyse par cytométrie en flux des cellules marquées à l'iodure de propidium indique, par ailleurs, que le co-traitement induit plus d'apoptose que l'irradiation seule, ou que le RDC-11 seul, et que le pourcentage de cellules en apoptose est supérieur à la somme des traitements pris séparément (figure 10).

Conclusions

Sur la base de ces deux critères et partant de ces résultats, on peut conclure que la combinaison RDC-11 + irradiation a un effet supra additif. Des résultats comparables ont été obtenus avec des rayons X utilisés par la radiothérapie, et des ions carbone. Ils ont été confirmés au moyen d'autres tests, tels que le MTT ou l'Alamar Blue.

***5. ANALYSE DES EFFETS CYTOSTATIQUES ET CYTOTOXIQUES DES COMPOSES DERIVES DU RUTHENIUM SUR DES CULTURES DE GLIOBLASTOMES ET NEUROBLASTOMES***

La première étape dans la caractérisation des effets anticancéreux des composés dérivés du ruthénium consiste à tester leur activité sur des lignées tumorales maintenues en culture et de comparer ces effets sur des lignées qui présentent des caractéristiques différentes de résistance aux traitements anticancéreux. Deux lignées de glioblastomes humaines (A172, HS683) et deux lignées de neuroblastomes (N2A et SH5Y) ont été utilisées afin de tester les effets cytostatiques des composés selon l'invention. Le cisplatine a été choisi comme agent cytotoxique de comparaison. En première approche, un test MTT a été utilisé pour mesurer l'activité d'une enzyme mitochondriale, ce qui donne une estimation du nombre de cellules. Par la suite, les effets cytotoxiques des composés selon l'invention ont été caractérisés plus finement par une analyse de la morphologie du noyau et de l'activation de la caspase 3, deux marqueurs de l'apoptose cellulaire.

***RESULTATS***

Plusieurs composés dérivés du ruthénium diminuent le nombre de cellules tumorales et sur l'ensemble des lignées testées, A172, HS683, N2A, et SH5Y (Figure 1, tableau 2). Pour les composés les plus actifs (composés **6, 9** et **12**), cet effet est observé à une concentration similaire ou inférieure à celle du cisplatine. En se basant sur ces expériences, il a été estimé des valeurs d'IC50 correspondant à la concentration nécessaire pour diminuer de moitié la quantité de cellules tumorales présentes par rapport à la condition contrôle. Ces résultats résumés dans le tableau 1 ont également été reproduits dans d'autres lignées cellulaires, les HCT116 et les 293, et sur des cultures primaires de glies.

Les analyses immunocytochimiques sur les cellules de glioblastome A172 ont montré qu'après 48 h de traitement les composés **6, 9** et **12** induisent une condensation et une fragmentation nucléaire caractéristique de l'apoptose (figure 2A, B). Par ailleurs, les cellules traitées avec les composés les plus actifs (composé **6**) présentent un marquage important du fragment actif de la caspase 3 (figure 2A, B). Ce résultat est renforcé par l'augmentation des niveaux protéiques du fragment actif de la caspase 3 détectées par Western blot dans les cellules A172 traitées au cisplatine ou à un composé (composé **6** ou **9,** figure 2C). Des résultats équivalents ont également été observés dans les cellules N2A et HCT116 (résultats non montrés).

Dans leur ensemble, les résultats montrent que les composés du ruthénium **(6, 9** et **12)** exercent des effets cytostatiques et cytotoxiques sur les lignées de neuroblastome, de glioblastome et d'autres types cellulaires. La caractérisation des effets cytotoxiques montre que ces CDR induisent l'apoptose dans ces divers types cellulaires, à l'instar de ce qui est observé avec le cisplatine.

***6. ANALYSE DES MECANISMES MOLECULAIRES MIS EN JEU PAR LES DERIVES DU RUTHENIUM :***

L'arrêt de la prolifération cellulaire ou l'induction de l'apoptose sont des processus cellulaires induits par diverses drogues anticancéreuses. Deux des protéines importantes dans le déclenchement de ces processus sont les protéines codées par les gènes homologues p53 et p73 (Marin and Kaelin 2000). Les protéines p53 et p73 codées par ces deux gènes sont des facteurs de transcription dont les niveaux protéiques sont induits en réponse à des dommages à l'ADN ou d'autres stress cellulaires. L'induction des niveaux protéiques de p53 passe par une augmentation de la stabilité de la protéine qui n'est plus dégradée par la voie du protéasome (Vargas, Takahashi et al. 2003; Yang, Li et al. 2004). Le cisplatine est un inducteur de p53 et de p73 (Siddik 2003). Par ailleurs, ces protéines, en tant que facteurs de transcription, induisent l'expression de gènes particuliers directement impliqués dans l'arrêt de la croissance cellulaire, comme p21 un inhibiteur des kinases dépendantes des cyclines, ou dans l'apoptose, comme bax qui est localisé dans la mitochondrie et participe au relargage du cytochrome C (Prives and Hall 1999).

Dans le but de déterminer les mécanismes moléculaires mis en jeu par les dérivés du ruthénium et de les comparer à ceux induits par le cisplatine, il a été analysé l'expression de p53, p73 p21 et bax dans les lignées A172 et HCT116.

***RESULTATS :***

Des analyses par Western blot ont été réalisées afin de déterminer si les CDR induisent les niveaux protéiques de p53 dans les cellules A172. Les résultats montrent des différences entre les CDR et le cisplatine. Après 24h de traitement, le cisplatine induit les niveaux protéiques de p53 mais aucun effet n'est observé pour le CDR **6** (figure 3). Par contre à 6 h de traitement, le CDR6 et le cisplatine induisent p53. Un résultat équivalent est observé sur les niveaux protéiques de p73.

Afin de déterminer si l'induction des protéines p53 et p73 conduit à l'activation des gènes cibles de ces protéines, l'expression de p21 et de Bax a été analysée par Western blot. Après 24 heures de traitement, le CDR **6** comme le cisplatine induisent l'expression de p21 et de Bax (figure 4).

Ces expériences ont été répétées dans les cellules HCT116 qui sont largement utilisées comme modèles d'étude de l'activation de p53 par des agents anticancéreux. Dans ces cellules, il a été également observé une induction de p53 par les CDR (CDR6), mais avec certaines différences (figure 5). D'une part, la cinétique d'activation est similaire à celle du cisplatine commençant à 6h et se maintenant jusqu'à 24h. D'autre part, les CDR induisent p53 plus faiblement que le cisplatine. Cependant, l'augmentation de l'expression de p21 et l'inhibition de la phosphorylation de l'histone H3, un marqueur de prolifération cellulaire, sont identiques entre le CDR **6** et le cisplatine.

Les données recueillies montrent que les CDR induisent des mécanismes moléculaires en partie identiques à ceux mis en jeu par le cisplatine (p53, p73, Bax, p21). Cependant, il existe aussi des différences qui indiquent que les CDR déclencheraient des mécanismes différents de ceux du cisplatine ou d'autres composés anticancéreux.

***7. ANALYSES DE LA SENSIBILITE DES DERIVES DU RUTHENIUM AUX MECANISMES CELLULAIRES DE RESISTANCE***

Les effets anticancéreux de drogues telles que le cisplatine sont malheureusement largement diminuées par le déclenchement par la cellule de processus de résistance qui bloquent des mécanismes apoptotiques (mutation de p53...) ou augmente l'expression de protéines qui détoxiquent la cellule. Pour le cisplatine, plusieurs mécanismes ont été décrits et ils sont également efficaces contre le carboplatine, un dérivé du cisplatine (Safaei, Katano et al. 2004). Il est donc particulièrement important de tester la sensibilité des CDR vis-à-vis de ces mécanismes de résistance afin de déterminer l'apport exact que peut avoir ces composés pour le traitement de tumeurs résistantes aux traitements chimiothérapeutiques déjà existants. L'un de ces mécanismes est la surexpression du la pompe d'export du cuivre (ATP7B) qui expulse le cisplatine hors de la cellule. Il a été montré que cette molécule est surexprimée dans des tumeurs humaines et que des lignées cellulaires qui surexpriment cette molécule sont plus résistantes au cisplatine et au carboplatine que des lignées contrôles.

***RESULTATS :***

Deux lignées cellulaires dérivées de cellules 2008 (Katano, Safaei et al. 2003) ont été étudiées. L'une surexprime la pompe ATP7B et l'autre ne contient que le vecteur contrôle. Il a été testé l'activité cytostatique du cisplatine et des CDR dans ces deux lignées en utilisant le test MTT. De manière très intéressante, il a été observé que la lignée exprimant ATP7B est significativement moins sensible au cisplatine. alors que sa sensibilité vis-à-vis du CDR6 est équivalente à celle de la lignée contrôle (figure 5).

Ces résultats suggèrent que les mécanismes de résistance développés par une cellule contre le cisplatine ou envers une autre drogue sont, en partie, moins efficace contre les CDR. Il est donc envisageable d'utiliser les CDR dans le traitement de tumeurs résistantes au cisplatine ou à d'autres drogues anticancéreuses.

***8. UTILISATION DES COMPOSES DERIVES DU RUTHENIUM EN COMBINAISON AVEC D'AUTRESAGENTS ANTICANCEREUX :***

Les mécanismes induits par diverses drogues anticancéreuses sont différents suivant le mode d'action de ces drogues. Depuis de nombreuses années des traitements anticancéreux combinant plusieurs drogues sont utilisées de manière à augmenter leur efficacité. En se basant sur cette approche, des traitements combinant des dérivés du ruthénium à d'autres drogues comme le NCS (Néocarzinostatin) et le taxol ont été entrepris et leur efficacité comparée à celle des traitements individuels. Le NCS induit des cassures double brin de l'ADN, similaires à celles des rayons gamma (Smith and Nicolaou 1996). Le taxol perturbe les fuseaux mitotiques (Oberlies and Kroll 2004).

***RESULTATS :***

Le NCS ou le Taxol sont des drogues avec un IC50 de l'ordre du nM. Une comparaison a été effectuée en

utilisant deux doses de NCS et deux doses de Taxol. L'une qui permet d'attendre l'IC50, l'autre qui conduit à une diminution de 10% de la viabilité cellulaire après 48 h de traitement. Ces deux concentrations ont été combinées avec une concentration de dérivé du ruthénium ou de cisplatine provoquant 10 % de l'effet maximal. Le co-traitement par les dérivés du ruthénium (CDR 6) est significativement plus efficace par rapport à un co-traitement avec le cisplatine (figure 6). Des résultats équivalents sont obtenus avec le taxol et le NCS.

Ces résultats montrent que les composés dérivés du ruthénium ont un effet « potentialisateur » significativement plus fort que le cisplatine sur l'activité d'autres drogues comme le NCS ou le taxol.

***9. TEST MTT:***

Les expériences sont réalisées sous une hotte à flux laminaire vertical. Le milieu de croissance cellulaire est composé de DMEM (Dulbecco's Modified Eagle's Medium), HEPES, 10% FCS (Fetal Calf Serum), 5% PS (Péniciline, Streptomycine) et est stocké à + 4 °C, ainsi que le PBS (Phosphate Buffer Saline, pH= 7,4). La Trypsine-EDTA (0,25 % trypsine dans 1 mM $Na_4$(EDTA)) et le FCS sont stockés à - 15 °C et décongelés avant utilisation. MTT (bromure de 4,5-dimethylthiazol-2-diphenyltetrazolium) est un solide jaune produit par la marque Aldrich. Il est mis en solution aqueuse stérile à la concentration de 5 mg/mL et conservé à + 4°C. Il est dilué à 10% dans du milieu de culture cellulaire lors de son utilisation comme agent de coloration de cellules vivantes. Les cellules humaines cancéreuses du colon (HCT-116) ou du foie (A-172) ont été achetées auprès de European Type Culture Collection, et placées dans un incubateur à 37 °C, 5% $CO_2$ dans des boîtes de Pétri rondes (diamètre 10 cm.) avec 10 mL de milieu. Quand elles sont assez nombreuses (confluence de 70%), elles sont lavées au PBS à température ambiante puis mélangées avec 1,5 mL de Trypsine-EDTA pour les décrocher de la boîte de Pétri. On les place à l'incubateur pendant quelques minutes pour accélérer ce détachement. Cette suspension cellulaire est placée dans du milieu de culture chauffé à 37 °C, puis cette solution est étalée dans des plaques de culture cellulaire de 96 puits (100 μL/puits) qu'on laisse incuber pendant 48 heures jusqu'à atteindre une confluence cellulaire de 50 %. Le milieu est renouvelé par du milieu cellulaire contenant différentes concentrations de RDCs et cisplatine à 37°C, qu'on laisse incuber. Au bout de 48 heures, le milieu est remplacé par une solution à 37 °C de MTT dans du milieu, qu'on place dans l'incubateur pendant au moins une heure ou jusqu'à ce que des cristaux violets issus de la complexation du MTT soient formés quantitativement au fond de chaque puits. Finalement, ce milieu est remplacé par 100 μL/puits d'une solution HCl/ $^{i}$PrOH 0,04 M à température ambiante pour dissoudre les cristaux. Une lecture de la densité optique des solutions obtenues est faite. On compare les densités optiques des puits traités aux RDCs ou cisplatine à celles des puits non traités (contrôles)

Une manipulation consiste à traiter 4 plaques (3 X RDC et le cisplatin). Chaque plaque contient un seul produit à différentes concentrations. 9 colonnes sont traitées à 50, 20, 15, 10, 7.5, 5, 2.5, 1 et 0.2 μM et 3 colonnes sont laissées comme contrôles. Seules les colonnes contrôles notées (i)m (1<i<4) (fig. 11) sont prises en compte pour les calculs.

La détermination de l'$IC_{50}$ et les tests statistiques de variance et Newmann-Keuls sont effectués à l'aide su logiciel Prism GraphPad v. 4.

Les résultats sur les MTT sont donnés à la figure 11 pour les HCT-116.

Un tableau récapitulatif des $IC_{50}$ obtenus est donné ci-dessous :

Tableau récapitulatif des $IC_{50}$

| $IC_{50}$ μM | A-172 | HCT-116 |
|---|---|---|
| RDC-17 | 5 | 5 - 7 |
| RDC-20 | 15 | 20 - 30 |
| RDC-24 | 10 | 1- 5 |
| RDC-28 | | |
| RDC-29 | 15 | |

***10. TEST IN VIVO:***

Le RDC-11 a été testé *in vivo* afin de confirmer ses propriétés anticancéreuses in *vitro* mises en évidence ci-dessus. Une expérience préliminaire sur un petit nombre de souris SWISS saines a montré l'absence de toxicité apparente de RDC-11.

Les animaux sont manipulés sous une hotte à flux laminaire. Dix souris SWISS nu/nu (athymiques) âgées de onze semaines, ont été greffées avec des cellules U-87 (un glioblastome humain) en sous-cutané au niveau de la cuisse gauche. Au bout de 7 jours, les tumeurs deviennent apparentes et le traitement commence. Les animaux sont séparés

en deux groupes de cinq, 5 souris sont ainsi traitées avec du D-PBS (souris contrôles) et les 5 autres avec du RDC-11 (souris traitées).

Pour les souris traitées, 2 mg de RDC-11 mis en solution dans 4 mL de D-PBS à chaud pour chaque injection. 0,5 mL de cette solution refroidie à température ambiante par voie intra-péritonéale est administré à chaque souris. Pour les souris contrôles, 0,5 mL de D-PBS à température ambiante est administré de la même façon.

Les jours d'injections sont marqués d'une croix dans le tableau ci-dessous :

mai-05

| Dim | Lun | Mar | Mer | Jeu | Ven | Sam |
|---|---|---|---|---|---|---|
| 22 | 23 | X 24 | 25 | X 26 | 27 | 28 |
| 29 | X 30 | 31 | | | | |

juin-05

| Dim | Lun | Mar | Mer | Jeu | Ven | Sam |
|---|---|---|---|---|---|---|
| | | | X 1 | 2 | X 3 | 4 |
| 5 | 6 | X 7 | X 8 | X 9 | X 10 | 11 |
| 12 | X 13 | 14 | S 15 | 16 | 17 | 18 |

X= jour d'injection

S= sacrifice des souris

La dose de RDC-11 injectée est de 18,5 mg/Kg de souris. La dose cumulée est donc de 185 mg/Kg.

L'évolution des tumeurs apparentes a été suivie en les mesurant avec un pied à coulisse, en pesant les souris et en scannant une souris de chaque lot par CT-scan après injection d'un produit de contraste (Fenestra VC). Les souris ont été individualisées par un marquage à l'oreille.

Le volume des tumeurs apparentes a été calculé selon la formule :

$$V= (4/3)*\pi*(L/2)*(l/2)^2$$

où L est la longueur et l la largeur mesurées.

Les figures 12 et 13 montrent l'évolution des volumes des tumeurs apparentes et des poids des souris en fonction du temps.

Lors de la dissection des souris, les tumeurs dans les deux groupes sont isolées et pesées (en g). Les résultats sont donnés à la figure 14. En comparant les figures des mesures des tumeurs (volume calculé et poids), il est possible de conclure que les volumes des tumeurs calculés est inférieur au volume des tumeurs observé. En effet, les tumeurs ont poussé en profondeur, d'où la difficulté de les mesurer avec un pied à coulisse. Néanmoins, le poids et le volume des tumeurs traitées sont inférieurs par rapport aux contrôles.

## Conclusions

RDC-11 infléchit la croissance tumorale, ceci en l'absence de toute toxicité.

***RÉFÉRENCES***

Alarcon-Vargas D. & Ronai Z. (2002). p53-Mdm2--the affair that never ends. Carcinogenesis. 23(4),541-7
Coelho D, Fischer B, Holl V, Jung GM, Dufour P, Bergerat JP, Denis JM, Gueulette J, Bischoff P. (2002). involvement of tp53 in apoptosis induced in human lymphoblastoid cells by fast neutrons. Radiat. Res. 157(4), 446-52
Katano, K., R Safaei, et al. (2003). "The copper export pump ATP7B modulates the cellular pharmacology of carboplatin in ovarian carcinoma cells." Mol Pharmacol 64(2): 466-73.
Marin, M. C. and W. G. Kaelin, Jr. (2000). "p63 and p73: old members of a new family." Biochim Biophys Acta 1470 (3): M93-M100.
Oberlies, N. H. and D. J. Kroll (2004). "Camptothecin and taxol: historic achievements in natural products research." J Nat Prod 67(2): 129-35.
Prives, C. and P. A. Hall (1999). "The p53 pathway." J Pathol 187(1): 112-26.
Safaei, R, K. Katano, et al. (2004). "Cross-resistance to cisplatin in cells with acquired resistance to copper." Cancer Chemother Pharmacol 53(3): 239-46.
Siddik, Z. H. (2003). "Cisplatin: mode of cytotoxic action and molecular basis of resistance." Oncogene 22(47): 7265-79.
Smith, A. L. and K. C. Nicolaou (1996). "The enediyne antibiotics." J Med Chem 39(11): 2103-17.
Vargas, D. A., S. Takahashi, et al. (2003). "Mdm2: A regulator of cell growth and death." Adv Cancer Res 89: 1-34.
Yang, Y., C. C. Li, et al. (2004). "Regulating the p53 system through ubiquitination." Oncogene 23(11): 2096-106.

**Revendications**

1. Composition pharmaceutique comprenant, dans un support acceptable sur le plan pharmaceutique, au moins un composé complexe du ruthénium (II) de formule générale (I) ou (II) suivante :

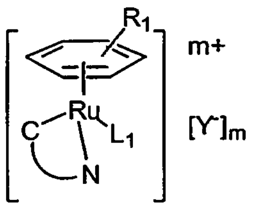

(I)

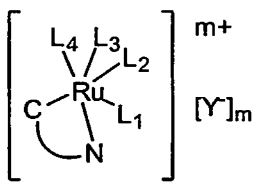

(II)

formule (I) ou (II) dans laquelle :

$L_1$, $L_2$, $L_3$ et $L_4$, identiques ou différents, représentent soit un ligand donneur de 2 électrons par un atome d'azote, d'oxygène, de phosphore ou de soufre, soit un atome d'halogène,
R1 représente un atome d'hydrogène ou une ou plusieurs substitutions sur le groupe phényle choisie parmi un radical (C1-6)alkyle et (C6-18)aryle,
Y est un contre-ion (lorsque m = 1),
m est 0 ou 1,
entre C et N, représentée par une ligne courbe, il existe une succession d'atomes formant, avec les atomes de carbone, d'azote et de ruthénium représentés sur les formules (I) et (II), le métallocycle, qui est constitué de 5 à 8 atomes (incluant les atomes de carbone, d'azote et de ruthénium représentés dans les formules (I) et (II)).

2. Composition selon la revendication précédente, **caractérisée en ce que** les ligands donneurs de deux électrons par un atome d'azote, d'oxygène, de phosphore ou de soufre sont choisis parmi $H_2O$, di(($C_{1-6}$)alkyl)O, di(($C_{1-6}$)alkyl)S, di(($C_{1-6}$)alkyl)S(O), ($C_{1-6}$)alkyl)$SO_3^-$, di(($C_{1-6}$)alkyl)C=O et ($C_{1-6}$)alkyl$CO_2^-$.

3. Composition selon la revendication 1, **caractérisée en ce que** les ligands donneurs de deux électrons par un atome d'azote sont choisis parmi les ligands de formule ($C_{1-6}$)alkylCN (en particulier $CH_3CN$) et des ligands pyridine, éventuellement substitués, sur un ou plusieurs atomes de carbone des cycles pyridine, par un radical ($C_{1-6}$)alkyle

ou un atome d'halogène.

4. Composition selon la revendication 1, **caractérisée en ce que** les ligands donneurs de deux électrons par un atome d'azote sont choisis parmi les amines primaires $(C_{1-6})$alkyle, tel que méthylamine ou éthylamine.

5. Composition selon la revendication 1, **caractérisée en ce que** les ligands donneurs de deux électrons par un atome de phosphore sont les ligands de type phosphine.

6. Composition selon la revendication 1, **caractérisée en ce que** les ligands donneurs de deux électrons par un atome de phosphore sont de formule $P(Ph)_{3-x}(alkyle)_x$, avec x représentant 0, 1 ou 2.

7. Composition selon l'une des revendications précédentes, **caractérisée en ce que** dans le cas de la formule (II), au moins deux des groupements $L_1$, $L_2$, $L_3$ et $L_4$, pris deux à deux, sont reliés par au moins une liaison covalente.

8. Composition selon l'une des revendications précédentes, **caractérisée en ce que** dans le cas de la formule (II), au moins deux des groupements $L_1$, $L_2$, $L_3$ et $L_4$, pris deux à deux, représentent les motifs bipyridine ou phénanthroline, éventuellement substitués, en particulier par au moins un radical alkyle, ou encore le 1,2-bisdiphénylephosphinoéthane.

9. Composition selon l'une des revendications précédentes, **caractérisée en ce que**, dans le cas de la formule (II), au moins un groupement $L_1$, $L_2$, $L_3$ et $L_4$, représentant un ligand donneur de deux électrons par un atome d'azote ou de phosphore, est un groupement pyridine, phosphine, bipyridine ou phénanthroline.

10. Composition selon l'une des revendications précédentes, **caractérisée en ce que**, dans le cas de la formule (II), au moins deux des groupements $L_1$, $L_2$, $L_3$ et $L_4$, représentent des ligands nitriles, comme par exemple des ligands de formule $(C_{1-6})$alkylCN (en particulier $CH_3CN$).

11. Composition selon l'une des revendications précédentes, **caractérisée en ce que,** dans le cas de la formule (II), deux des groupements $L_1$, $L_2$, $L_3$ et $L_4$, représentent des ligands nitriles, comme par exemple des ligands de formule $(C_{1-6})$alkylCN (en particulier $CH_3CN$), et les deux autres ligands sont reliés par au moins une liaison covalente.

12. Composition selon l'une des revendications précédentes, **caractérisée en ce que** Y est $PF_6^-$, $BF_4^-$, $CF_3CO_2^-$, $CH_3CO_2^-$, $CF_3SO_3^-$, ou $NO_3^-$.

13. Composition selon l'une des revendications précédentes, **caractérisée en ce que** m est égal à 1.

14. Composition selon l'une des revendications précédentes, **caractérisée en ce que** la ligne courbe est un métallocycle à 5 atomes choisi parmi un métallocycle défini selon l'une des formules suivantes :

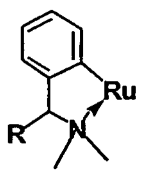

R = H, Me

(1)

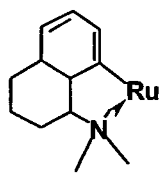

(2)

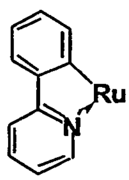

(3)

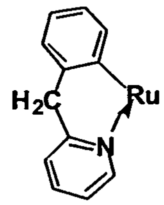

(4)

15. Composition selon l'une des revendications précédentes, **caractérisée en ce que** la ligne courbe est un métallocycle à 6 ou 7 atomes choisi parmi un métallocycle défini selon l'une des formules suivantes :

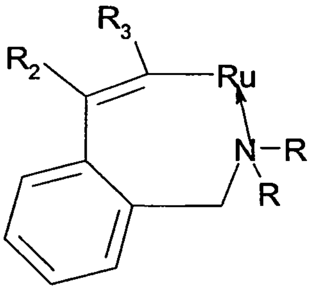
(5)

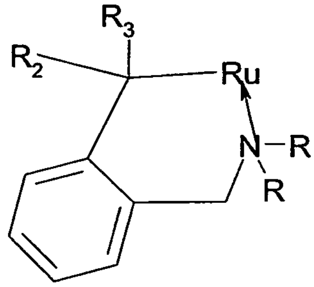
(6)

avec R, identique ou différent, représentant H ou un radical alkyle, de préférence méthyle, et $R_2$ et $R_3$, identiques ou différents, représentant un atome d'hydrogène, un atome d'halogène, un groupe alkyle, un radical alkoxy, thiol, thioether, hydroxyl, nitro, cyano ou ester.

**16.** Composition selon la revendication 1, **caractérisée en ce que** le composé est choisi parmi les composés 3 à 6 et 8 à 29.

**17.** Composé du ruthénium, choisi parmi les composés suivants :

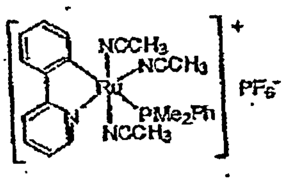
9

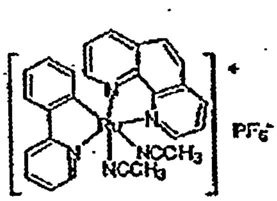
11

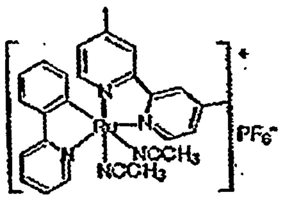
12

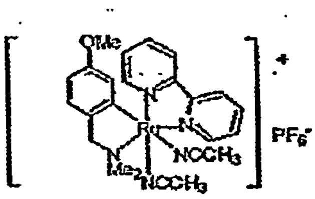
14

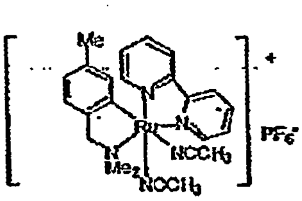
15

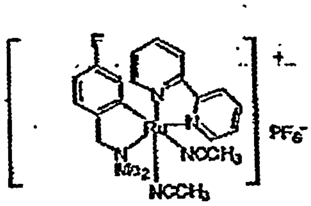
16

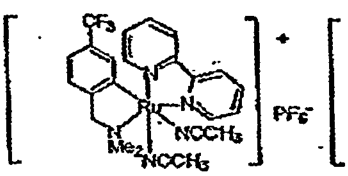
17

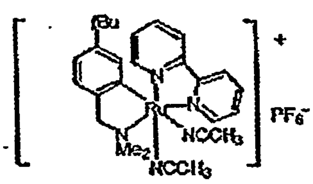
18

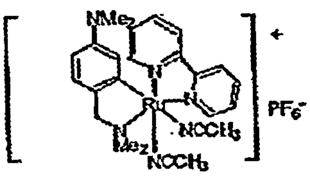
19

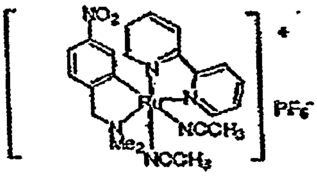

20

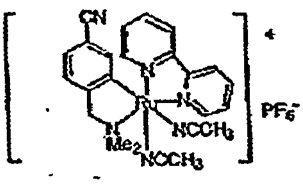

21

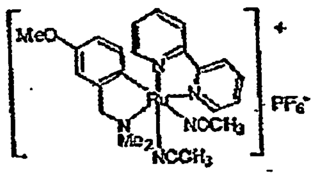

22

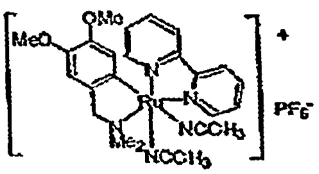

23

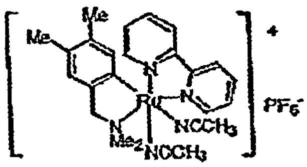

24

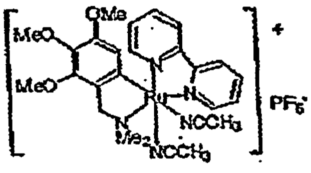

25

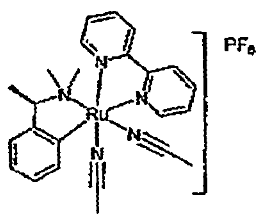

26

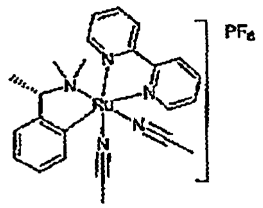

27

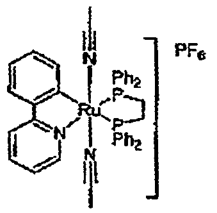

28

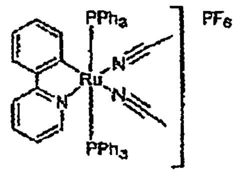

29

**18.** Composition selon l'une des revendications 1 à 16, pour son utilisation pour le traitement des maladies liées à une hyperprolifération cellulaire.

**19.** Composition selon l'une des revendications 1 à 16, pour son utilisation pour le traitement des cancers.

**20.** Composition selon l'une des revendications 1 à 16, pour son utilisation pour le traitement des glioblastomes, des leucémies (promyélocytaires), des cancers de la prostate, des ovaires, des poumons, des seins, des voies digestives, en particulier du foie, du pancréas, de la tête et du cou, du colon, de la vessie, des lymphomes non-Hodgkiniens ou des mélanomes.

**21.** Composition selon l'une des revendications 1 à 16, pour son utilisation pour le traitement des cancers par accumulation des cellules tumorales en phase G0/G1, et éventuellement par induction d'apoptose.

**22.** Composition selon l'une des revendications 1 à 16, pour son utilisation pour le traitement des tumeurs résistantes au cisplatine ou à d'autres drogues anticancéreuses.

**23.** Composition selon l'une des revendications 1 à 16, pour son utilisation pour le traitement des cancers en combinaison avec un traitement anti-cancéreux mettant en oeuvre des rayonnements, tel que la radiothérapie et la curiethérapie.

**24.** Composition selon l'une des revendications 1 à 16, pour son utilisation pour le traitement des cancers en combinaison avec au moins un autre agent chimique anti-cancéreux, conditionné et administré de façon combinée, séparée ou séquentielle.

**25.** Composés selon la revendication 17, à titre de médicaments.

## Claims

**1.** A pharmaceutical composition comprising, in a pharmaceutically acceptable support, at least one complex ruthenium compound (II) with the following general formula (I) or (II) :

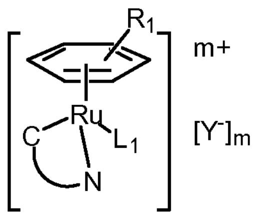

(I)

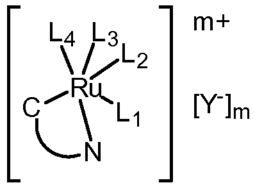

(II)

formula (I) or (II) in which :

$L_1$, $L_2$, $L_3$ and $L_4$, identical or different, represent either a donor ligand with 2 electrons to one nitrogen, oxygen, phosphorus or sulphur atom, or a halogen atom,
R1 represents a hydrogen atom or one or more substitutions on the phenyl group, selected from a (C1-6)alkyl and (C6-18)aryl radical,
Y is a counter-ion (when m = 1),
m is 0 or 1,
between C and N, represented by a curved line, there is a series of atoms forming, with the carbon, nitrogen and ruthenium atoms shown in formulae (I) and (II), the metallocycle, which is formed by between 5 and 8 atoms (including the carbon, nitrogen and ruthenium atoms shown in formulae (I) and (II)).

**2.** The composition according to the preceding claim, **characterised in that** the donor ligands with two electrons to one nitrogen, oxygen, phosphorus or sulphur atom are selected from $H_2O$, di(($C_{1-6}$)alkyl)O, di(($C_{1-6}$)alkyl)S, di(($C_{1-6}$)alkyl)S(O), ($C_{1-6}$)alkyl)$SO_3^-$, di(($C_{1-6}$)alkyl)C=O et ($C_{1-6}$)alkyl$CO_2^-$.

**3.** The composition according to Claim 1, **characterised in that** the donor ligands with two electrons to one nitrogen atom are selected from the ligands with the formula ($C_{1-6}$)alkylCN (in particular $CH_3CN$) and pyridine ligands, possibly substituted, on one or more carbon atoms from the pyridine cycles, by a ($C_{1-6}$)alkyl radical or a halogen atom.

**4.** The composition according to Claim 1, **characterised in that** the donor ligands with two electrons to one nitrogen atom are selected from the primary ($C_{1-6}$)alkyl amines such as methylamine or ethylamine.

**5.** The composition according to Claim 1, **characterised in that** the donor ligands with two electrons to one phosphorous atom are ligands of the phosphine type.

**6.** The composition according to Claim 1, **characterised in that** the donor ligands with two electrons to one phosphorus atom have the formula
$P(Ph)_{3-x}(alkyl)_x$, with x representing 0, 1 or 2.

**7.** The composition according to any of the preceding claims, **characterised in that** in the case of formula (II), at least two of the $L_1$, $L_2$, $L_3$ and $L_4$ groups, taken two by two, are linked by at least one covalent bond.

**8.** The composition according to any of the preceding claims, **characterised in that** in the case of formula (II), at least two of the $L_1$, $L_2$, $L_3$ and $L_4$ groups, taken two by two, represent the bipyridine or phenanthroline motifs, possibly substituted, in particular by at least one alkyl radical, or else by 1,2-bisdiphenylphosphinoethane.

**9.** The composition according to any of the preceding claims, **characterised in that**, in the case of formula (II), at least one $L_1$, $L_2$, $L_3$ and $L_4$ group representing a donor ligand with two electrons to one nitrogen or phosphorus atom, is a pyridine, phosphine, bipyridine or phenanthroline group.

**10.** The composition according to any of the preceding claims, **characterised in that**, in the case of formula (II), at least two of the $L_1$, $L_2$, $L_3$ and $L_4$ groups represent nitrile ligands, such as, for example, ligands with the formula $(C_{1-6})$alkylCN (in particular $CH_3CN$).

**11.** The composition according to any of the preceding claims, **characterised in that**, in the case of formula (II), two of the $L_1$, $L_2$, $L_3$ and $L_4$ groups represent nitrile ligands, such as for example ligands with the formula $(C_{1-6})$alkylCN (in particular $CH_3CN$), and the two other ligands are linked by at least one covalent bond.

**12.** The composition according to any of the preceding claims, **characterised in that** $Y^-$ is $PF_6^-$, $BF_4^-$, $CF_3CO_2^-$, $CH_3CO_2^-$, $CF_3SO_3^-$, or $NO_3^-$.

**13.** The composition according to any of the preceding claims, **characterised in that** m is equal to 1.

**14.** The composition according to any of the preceding claims, **characterised in that** the curved line is a metallocycle with 5 atoms selected from a metallocycle defined according to any of the following formulae :

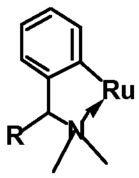

R = H, Me

(1)

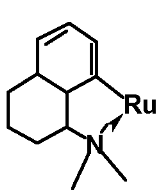

(2)

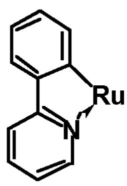

(3)

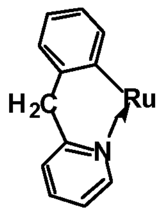

(4)

**15.** The composition according to any of the preceding claims, **characterised in that** the curved line is a metallocycle with 6 or 7 atoms selected from a metallocycle defined according to either of the following formulae :

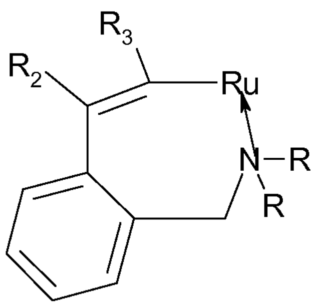

(5)

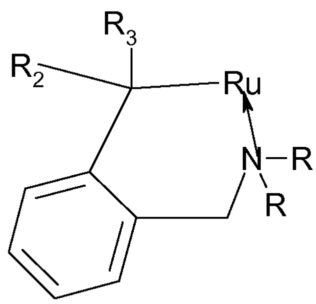

(6)

with R, identical or different, representing H or an alkyl radical, preferably methyl, and $R_2$ and $R_3$, identical or different, representing a hydrogen atom, a halogen atom, an alkyl group, an alkoxy, thiol, thioether, hydroxyl, nitro, cyano or ester radical.

**16.** The composition according to Claim 1, **characterised in that** the compound is selected from compounds 3 to 6 and 8 to 29.

**17.** A ruthenium compound, selected from the following compounds :

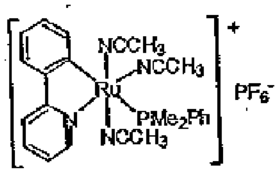

9

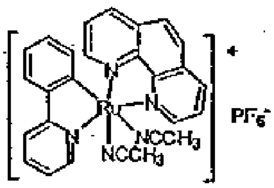

11

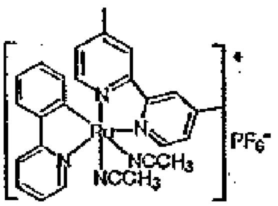

12

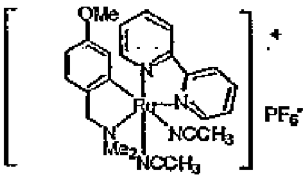

14

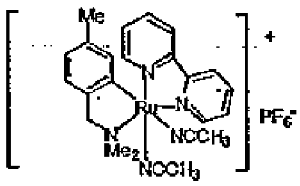

15

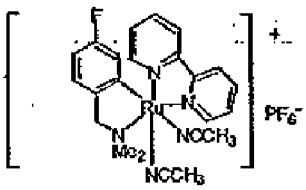

16

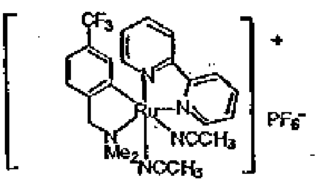

17

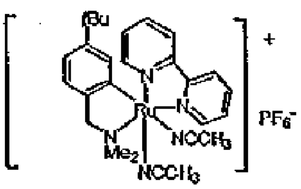

18

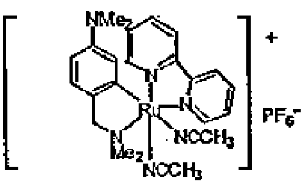

19

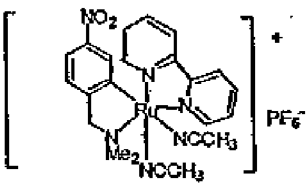

20

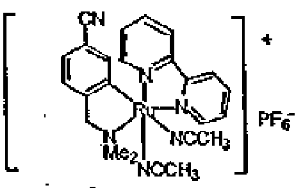

21

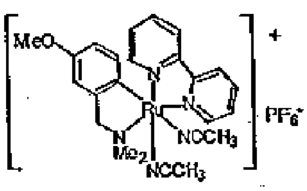

22

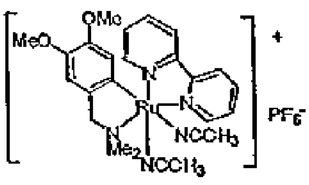

23

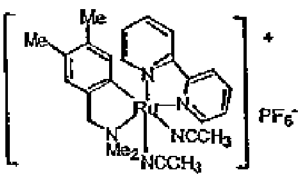

24

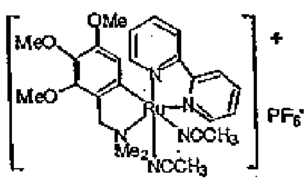

25

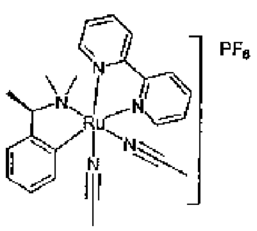

26

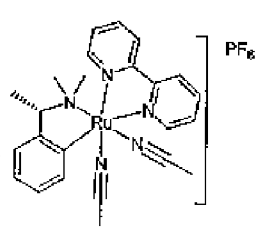

27

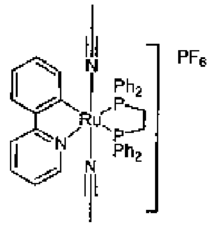

28

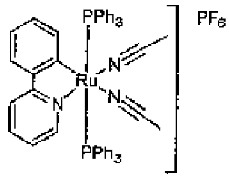

29

**18.** The composition according to any of Claims 1 to 16, for use in treatment of diseases linked to cell hyperproliferation.

**19.** The composition according to any of Claims 1 to 16, for use in treatment of cancers.

**20.** The composition according to any of Claims 1 to 16, for use in treatment of glioblastomas, (promyelocytary) leukemias, cancers of the prostate, the ovaries, the lungs, the breasts, the digestive tract, in particular of the liver, of the pancreas, of the head and of the neck, of the colon, of the bladder, non-Hodgkin lymphomas or melanomas.

**21.** The composition according to any of Claims 1 to 16, for use in treatment of cancers by accumulating the tumoral cells in the G0/G1 phase, and possibly by inducing apoptosis.

**22.** The composition according to any of Claims 1 to 16, for use in treatment of tumours which are resistant to cisplatinum or to other anticancer drugs.

**23.** The composition according to any of Claims 1 to 16, for use in treatment of cancers in combination with an anti-cancer treatment implementing radiation, such as radiotherapy and brachytherapy.

**24.** The composition according to any of Claims 1 to 16, for use in treatment of cancers in combination with at least one other chemical anti-cancer agent, conditioned and administered in combination, separately or sequentially.

**25.** The compounds according to Claim 17, as drugs.

## Patentansprüche

**1.** Pharmazeutische Zusammensetzung, umfassend in einem pharmazeutisch verträglichen Träger wenigstens eine Ruthenium(II)-Komplexverbindung mit der folgenden allgemeinen Formel (I) oder (II):

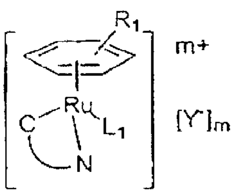

(I)

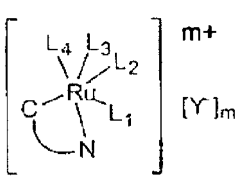

(II)

Formel (I) oder (II), in der:

$L_1$, $L_2$, $L_3$ und $L_4$ identisch oder verschieden sind und entweder für einen Donor-Liganden mit 2 Elektronen von einem Stickstoff-, Sauerstoff-, Phosphor- oder Schwefelatom oder für ein Halogenatom stehen,
$R_1$ für ein Wasserstoffatom oder eine oder mehrere Substitutionen an der Phenylgruppe steht, die aus einem

(C1-6)Alkyl- und (C6-18)Aryl-Rest ausgewählt sind,
Y ein Gegenion ist (wenn m = 1),
m 0 oder 1 ist,
zwischen C und N, mit einer gekrümmten Linie dargestellt, eine Reihe an Atomen vorhanden sind, die mit den in den Formeln (I) und (II) dargestellten Kohlenstoff-, Stickstoff- und Rutheniumatomen den Metallocyclus bilden, der aus 5 bis 8 Atomen besteht (einschließlich der in Formel (I) und (II) dargestellten Kohlenstoff-, Stickstoff- und Rutheniumatome).

2. Zusammensetzung gemäß dem vorhergehenden Anspruch, **dadurch gekennzeichnet, dass** die Donor-Liganden mit zwei Elektronen von einem Stickstoff-, Sauerstoff-, Phosphor- oder Schwefelatom aus $H_2O$, Di(($C_{1-6}$)alkyl)O, Di(($C_{1-6}$)alkyl)S, Di(($C_{1-6}$)alkyl)S(O), ($C_{1-6}$)Alkyl-$SO_3^-$, Di(($C_{1-6}$)alkyl)C=O und ($C_{1-6}$)Alkyl-$CO_2^-$ ausgewählt sind.

3. Zusammensetzung gemäß Anspruch 1, **dadurch gekennzeichnet, dass** die Donor-Liganden mit zwei Elektronen von einem Stickstoffatom aus den Liganden der Formel ($C_{1-6}$)Alkyl-CN (insbesondere $CH_3CN$) und Pyridin-Liganden ausgewählt sind, die gegebenenfalls an einem oder mehreren Kohlenstoffatomen der Pyridinringe mit einem ($C_{1-6}$) Alkyl-Rest oder einem Halogenatom substituiert sind.

4. Zusammensetzung gemäß Anspruch 1, **dadurch gekennzeichnet, dass** die Donor-Liganden mit zwei Elektronen von einem Stickstoffatom aus den primären ($C_{1-6}$)Alkylaminen, wie Methylamin oder Ethylamin, ausgewählt sind.

5. Zusammensetzung gemäß Anspruch 1, **dadurch gekennzeichnet, dass** die Donor-Liganden mit zwei Elektronen von einem Phosphoratom Liganden des Phosphin-Typs sind.

6. Zusammensetzung gemäß Anspruch 1, **dadurch gekennzeichnet, dass** die Donor-Liganden mit zwei Elektronen von einem Phosphoratom die Formel $P(Ph)_{3-x}(Alkyl)_x$ besitzen, wobei x für 0, 1 oder 2 steht.

7. Zusammensetzung gemäß einem der vorhergehenden Ansprüche, **dadurch gekennzeichnet, dass** im Fall der Formel (II) wenigstens zwei der $L_1$, $L_2$, $L_3$ und $L_4$ Gruppen paarweise vorliegen und mit wenigstens einer kovalenten Bindung verbunden sind.

8. Zusammensetzung gemäß einem der vorhergehenden Ansprüche, **dadurch gekennzeichnet, dass** im Fall der Formel (II) wenigstens zwei der $L_1$, $L_2$, $L_3$ und $L_4$ Gruppen paarweise vorliegen und für Bipyridin- oder Phenanthrolin-Einheiten, die gegebenenfalls insbesondere mit wenigstens einem Alkyl-Rest substituiert sind, oder auch für 1,2-Bis(diphenylphosphino)ethan stehen.

9. Zusammensetzung gemäß einem der vorhergehenden Ansprüche, **dadurch gekennzeichnet, dass** im Fall der Formel (II) wenigstens eine $L_1$, $L_2$, $L_3$ und $L_4$ Gruppe, die für einen Donor-Liganden mit zwei Elektronen von einem Stickstoff- oder Phosphoratom steht, eine Pyridin-, Phosphin-, Bipyridin- oder Phenanthrolin-Gruppe ist.

10. Zusammensetzung gemäß einem der vorhergehenden Ansprüche, **dadurch gekennzeichnet, dass** im Fall der Formel (II) wenigstens zwei der $L_1$, $L_2$, $L_3$ und $L_4$ Gruppen für Nitril-Liganden stehen, wie zum Beispiel Liganden der Formel ($C_{1-6}$)Alkyl-CN (insbesondere $CH_3CN$).

11. Zusammensetzung gemäß einem der vorhergehenden Ansprüche, **dadurch gekennzeichnet, dass** im Fall der Formel (II) wenigstens zwei der $L_1$, $L_2$, $L_3$ und $L_4$ Gruppen für Nitril-Liganden stehen, wie zum Beispiel Liganden der Formel ($C_{1-6}$)Alkyl-CN (insbesondere $CH_3CN$), und die beiden anderen Liganden mit wenigstens einer kovalenten Bindung verbunden sind.

12. Zusammensetzung gemäß einem der vorhergehenden Ansprüche, **dadurch gekennzeichnet, dass** $Y^-$ $PF_6^-$, $BF_4^-$, $CF_3CO_2^-$, $CH_3CO_2^-$, $CF_3SO_3^-$ oder $NO_3^-$ ist.

13. Zusammensetzung gemäß einem der vorhergehenden Ansprüche, **dadurch gekennzeichnet, dass** m gleich 1 ist.

14. Zusammensetzung gemäß einem der vorhergehenden Ansprüche, **dadurch gekennzeichnet, dass** die gekrümmte Linie ein Metallocyclus mit 5 Atomen ist, der aus einem gemäß einer der folgenden Formeln definierten Metallocyclus ausgewählt ist:

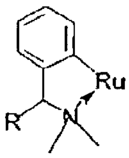

R = H, Me

(1)

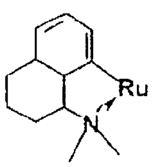

(2)

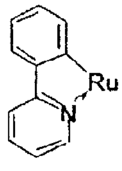

(3)

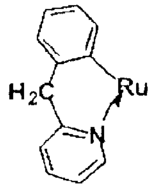

(4)

**15.** Zusammensetzung gemäß einem der vorhergehenden Ansprüche, **dadurch gekennzeichnet, dass** die gekrümmte Linie ein Metallocyclus mit 6 oder 7 Atomen ist, der aus einem gemäß einer der folgenden Formeln definierten Metallocyclus ausgewählt ist:

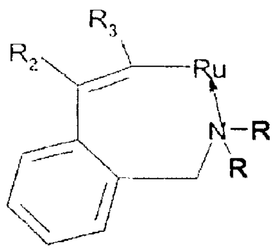

(5)

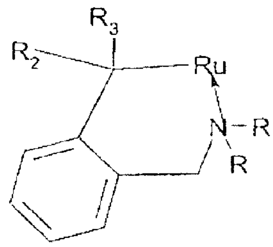

(6)

wobei R identisch oder verschieden ist und für H oder einen Alkyl-Rest, vorzugsweise Methyl, steht, und $R_2$ und $R_3$ identisch oder verschieden sind und für ein Wasserstoffatom, ein Halogenatom, eine Alkyl-Gruppe, einen Alkoxy-, Thiol-, Thioether-, Hydroxyl-, Nitro-, Cyan- oder Ester-Rest stehen.

**16.** Zusammensetzung gemäß Anspruch 1, **dadurch gekennzeichnet, dass** die Verbindung aus den Verbindungen 3 bis 6 und 8 bis 29 ausgewählt ist.

**17.** Rutheniumverbindung, die aus den folgenden Verbindungen ausgewählt ist:

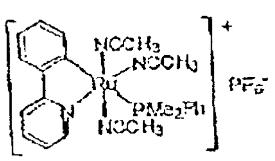

9

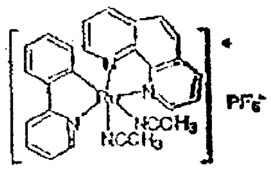

11

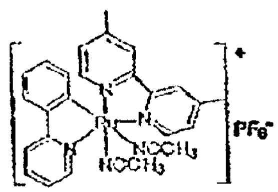

12

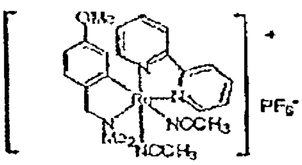

14

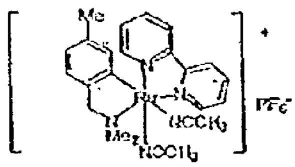

15

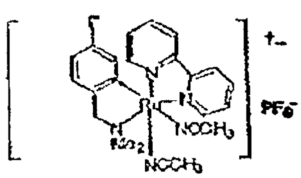

16

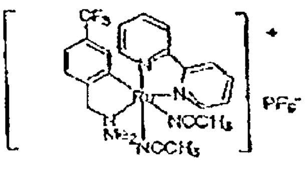

17

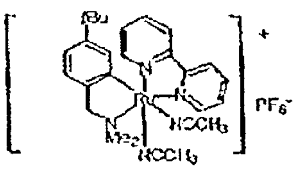

18

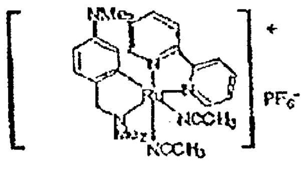

19

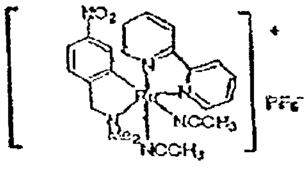

20

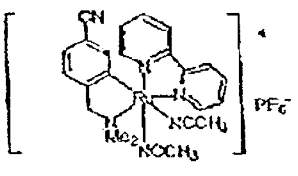

21

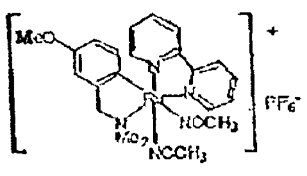

22

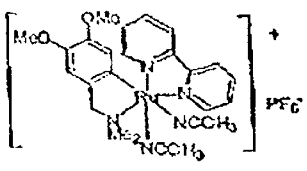

23

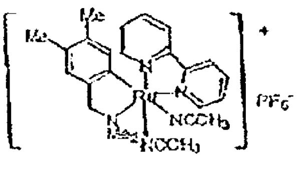

24

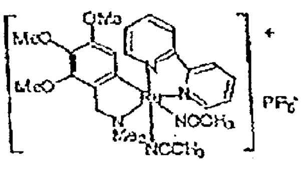

25

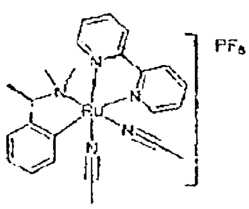

26

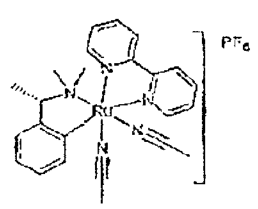

27

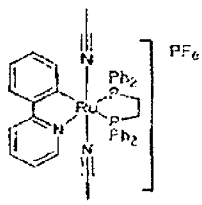

28

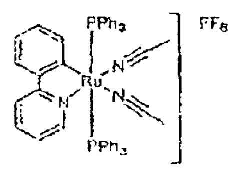

29

**18.** Zusammensetzung gemäß einem der Ansprüche 1 bis 16 für die Verwendung zur Behandlung von Krankheiten, die mit einer Zellhyperproliferation verbunden sind.

**19.** Zusammensetzung gemäß einem der Ansprüche 1 bis 16 für die Verwendung zur Behandlung von Krebserkrankungen.

**20.** Zusammensetzung gemäß einem der Ansprüche 1 bis 16 für die Verwendung zur Behandlung von Glioblastomen, (promyelozytären) Leukämien, Krebserkrankungen der Prostata, der Ovarien, der Lunge, der Brust, der Verdauungswege, insbesondere der Leber, des Pankreas, des Kopfes oder des Halses, des Darms, der Blase, Non-Hodgkin-Lymphomen oder Melanomen.

**21.** Zusammensetzung gemäß einem der Ansprüche 1 bis 16 für die Verwendung zur Behandlung von Krebserkrankungen durch Akkumulation von Tumorzellen in der G0/G1-Phase und gegebenenfalls durch Apoptose-Induktion.

**22.** Zusammensetzung gemäß einem der Ansprüche 1 bis 16 für die Verwendung zur Behandlung von Tumoren, die gegen Cisplatin oder andere Krebsmedikamente resistent sind.

**23.** Zusammensetzung gemäß einem der Ansprüche 1 bis 16 für die Verwendung zur Behandlung von Krebserkrankungen in Kombination mit einer Krebsbehandlung mit Hilfe von Bestrahlungen, wie Radiotherapie und Curie-Therapie.

**24.** Zusammensetzung gemäß einem der Ansprüche 1 bis 16 für die Verwendung zur Behandlung von Krebserkrankungen in Kombination mit wenigstens einem weiteren Krebschemotherapeutikum, das kombiniert, separat oder nacheinander konditioniert und verabreicht wird.

**25.** Verbindungen gemäß Anspruch 17 als Medikamente.

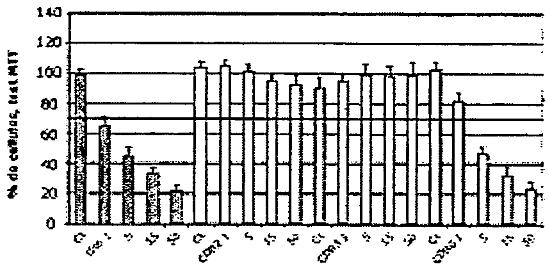


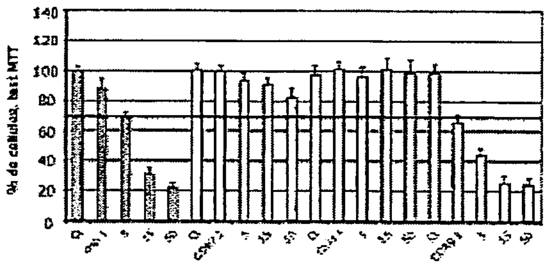


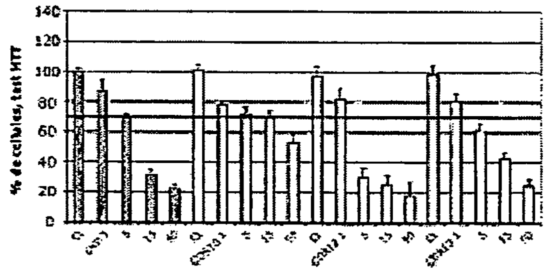


| | A172 | HS683 | N2A | SH5Y | HCT116 | 293 | Glies |
|---|---|---|---|---|---|---|---|
| Cisp | 1-5 | 1-5 | 1-5 | 1-5 | 1-5 | 1-5 | 1-5 |
| CDR2 | >50 | >50 | >50 | >50 | >50 | >50 | >50 |
| CDR3 | >50 | >50 | >50 | >50 | >50 | >50 | >50 |
| CDR6 | 1-5 | 1-5 | 1-5 | 1-5 | 1-5 | 1-5 | 5-15 |
| CDR7 | >50 | >50 | >50 | >50 | >50 | >50 | >50 |
| CDR8 | >50 | >50 | >50 | >50 | >50 | >50 | >50 |
| CDR9 | 1-5 | 1-5 | 1-5 | 1-5 | 1-5 | 1-5 | 5-15 |
| CDR10 | >50 | >50 | >50 | >50 | >50 | >50 | >50 |
| CDR12 | 1-5 | 1-5 | 1-5 | 1-5 | 1-5 | 1-5 | 1-5 |
| CDR13 | 5-15 | 5-15 | 5-15 | 5-15 | 5-15 | 5-15 | >50 |

Tableau 2

FIGURE 1

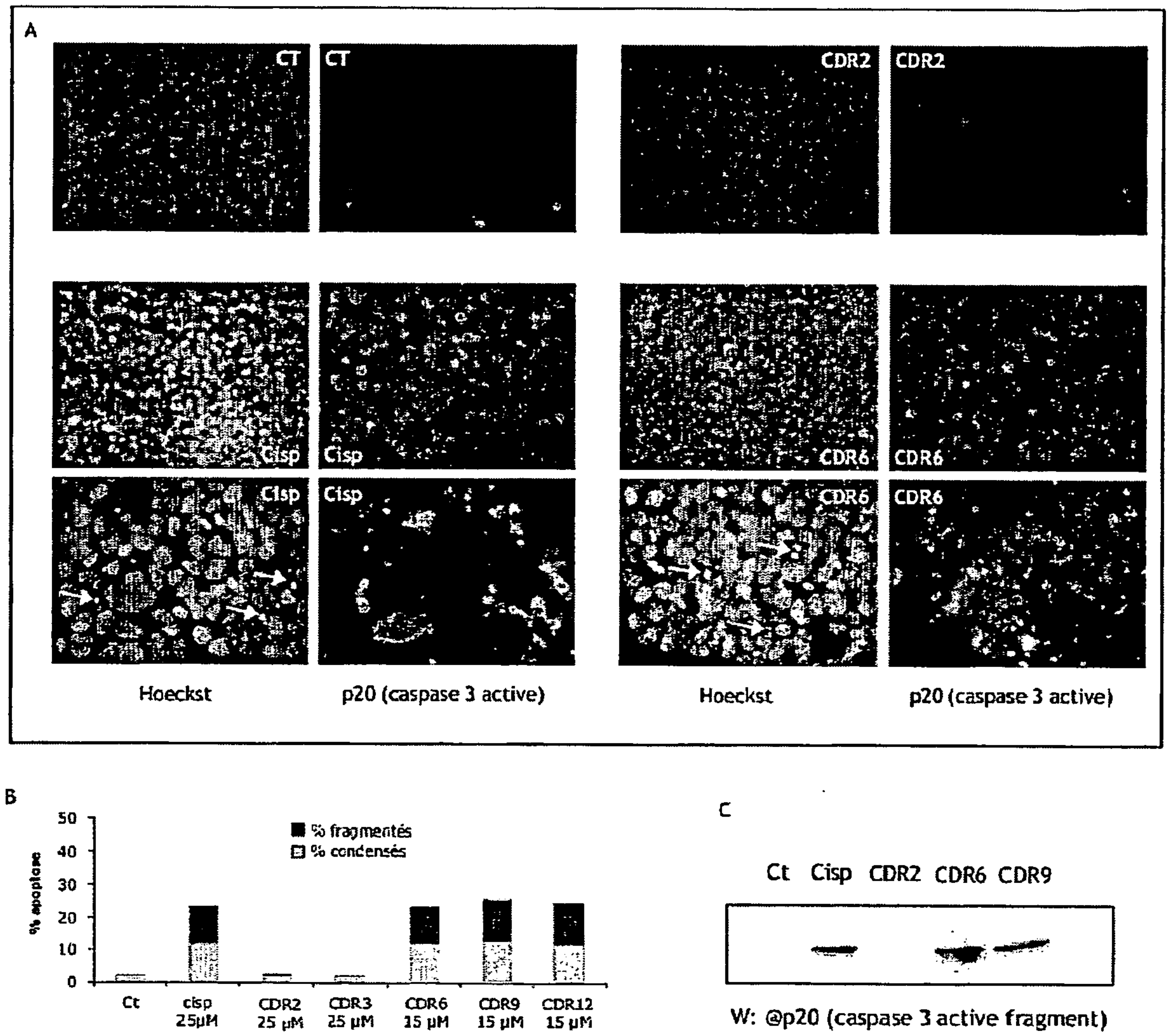
A
CT
CT
CDR2
CDR2
Cisp
Cisp
CDR6
CDR6
Cisp
Cisp
CDR6
CDR6
Hoeckst
p20 (caspase 3 active)
Hoeckst
p20 (caspase 3 active)
B
% apoptose
% fragmentés
% condensés
0
10
20
30
40
50
Ct
cisp 25µM
CDR2 25 µM
CDR3 25 µM
CDR6 15 µM
CDR9 15 µM
CDR12 15 µM
C
Ct Cisp CDR2 CDR6 CDR9
W: @p20 (caspase 3 active fragment)

FIGURE 2

Cellules A 172

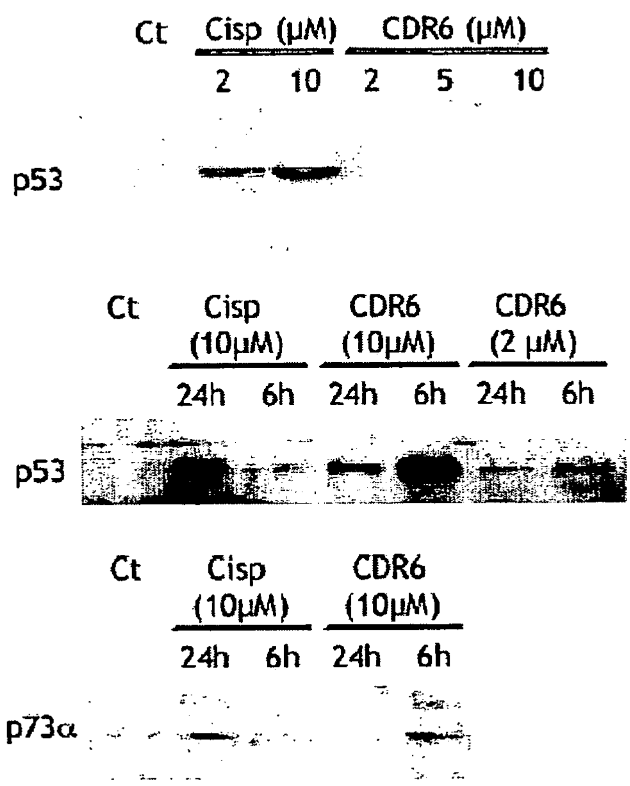


FIGURE 3

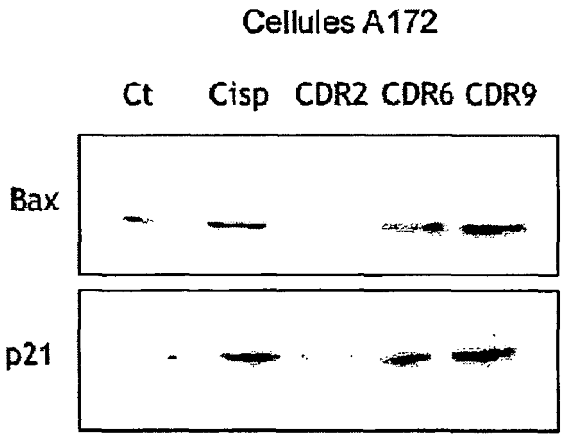


FIGURE 4

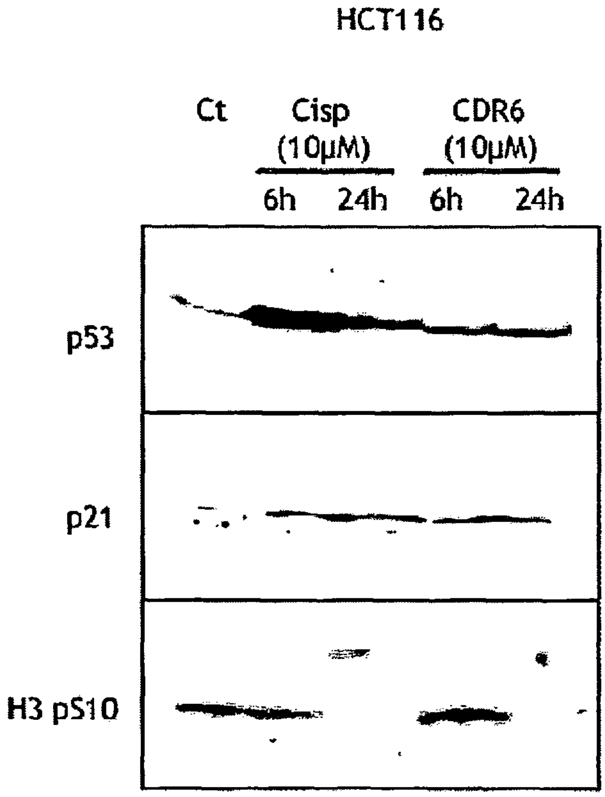


FIGURE 5

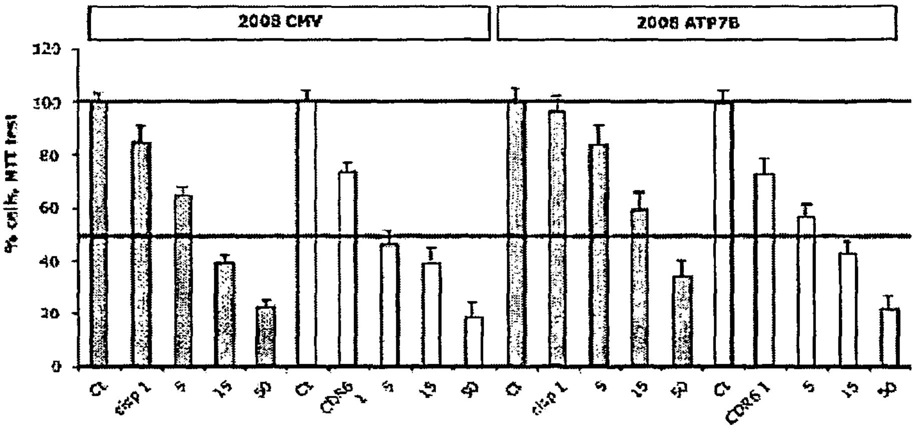
2008 CMV
2008 ATP7B
% cells, MTT test
120
100
80
60
40
20
0
Ct
5
15
50
Ct
5
15
50
Ct
5
15
50
Ct
CDR6 1
5
15
50

FIGURE 6

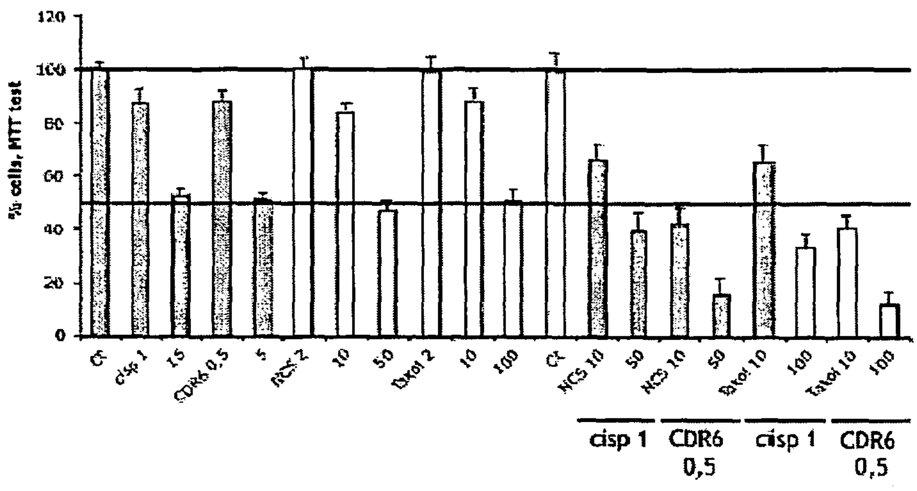

% cells, MTT test
120
100
80
60
40
20
0
Ct
cisp 1
15
CDR6 0,5
5
NCS 2
10
50
Taxol 2
10
100
Ct
NCS 10
50
NCS 10
50
Taxol 10
100
Taxol 10
100
cisp 1
CDR6 0,5
cisp 1
CDR6 0,5


FIGURE 7

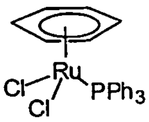

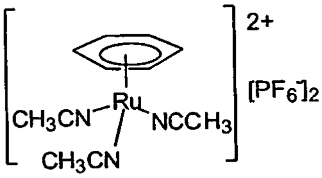

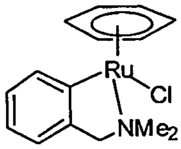

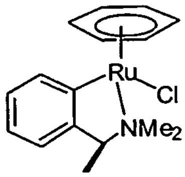

1 2 3 4

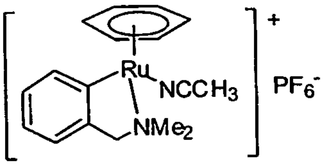

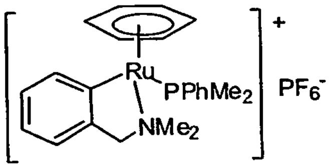

5 6

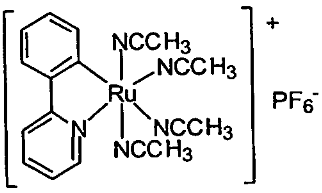

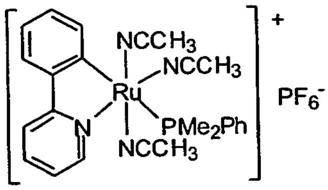

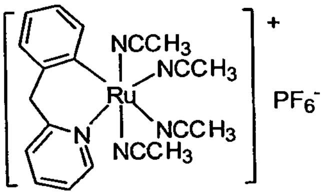

8 9 10

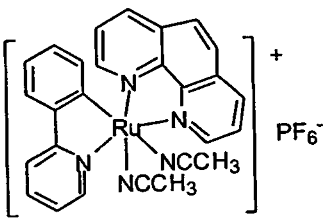

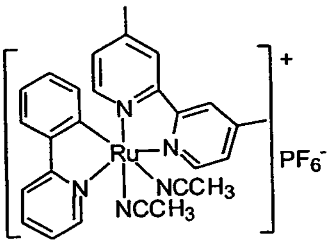

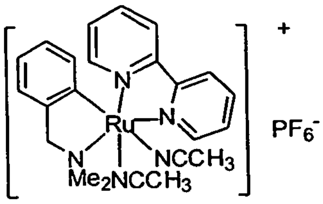

11 12 13

FIGURE 8

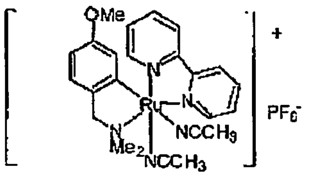

14

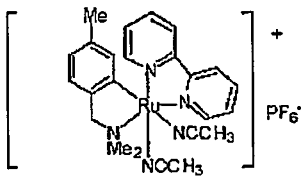

15

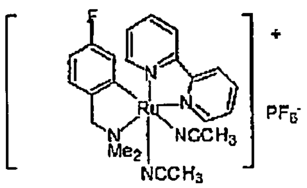

16

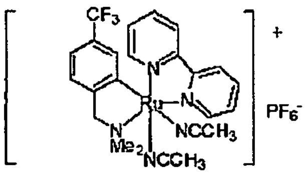

17

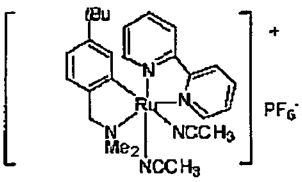

18

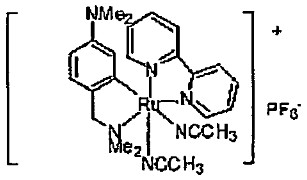

19

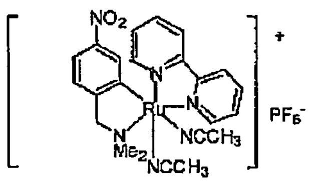

20

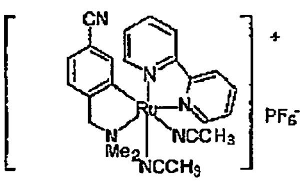

21

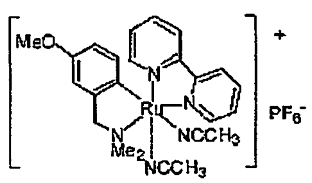

22

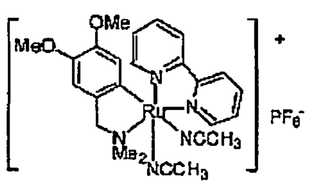

23

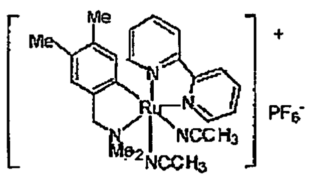

24

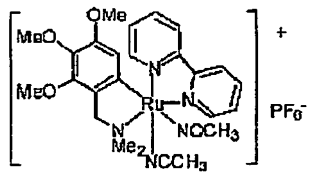

25

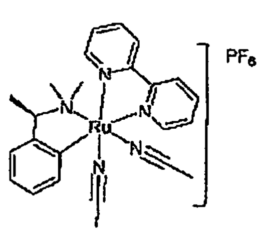

26

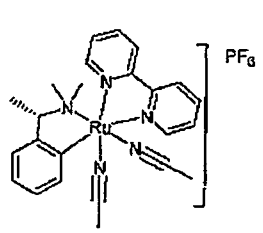

27

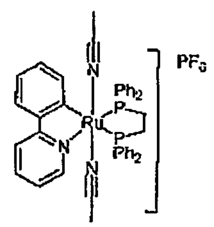

28

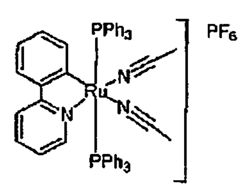

29

FIGURE 8 (suite)

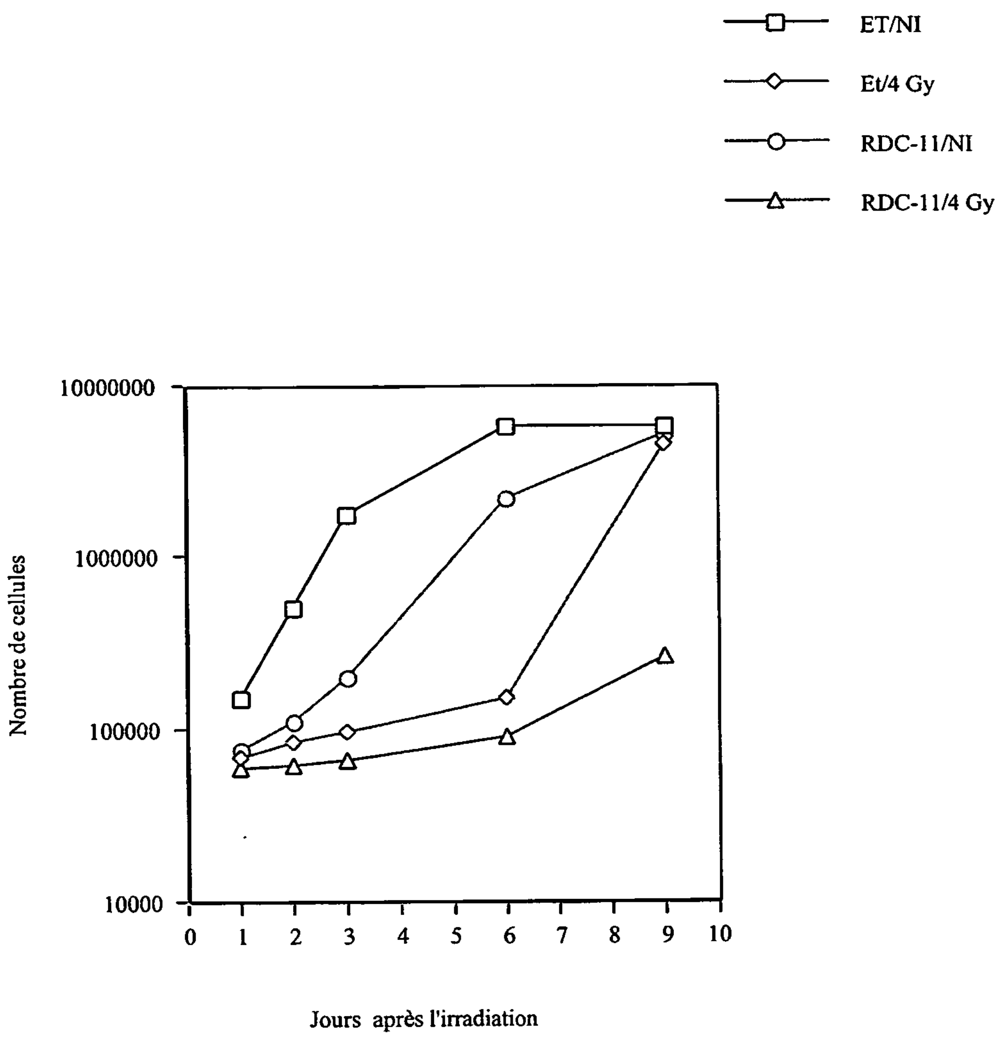
ET/NI
Et/4 Gy
RDC-11/NI
RDC-11/4 Gy
10000000
1000000
100000
10000
Nombre de cellules
0
1
2
3
4
5
6
7
8
9
10
Jours après l'irradiation

FIGURE 9

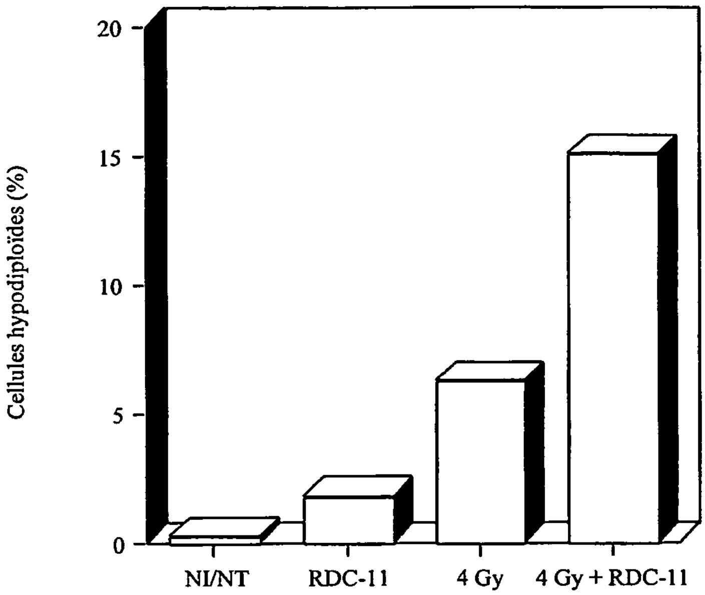


FIGURE 10

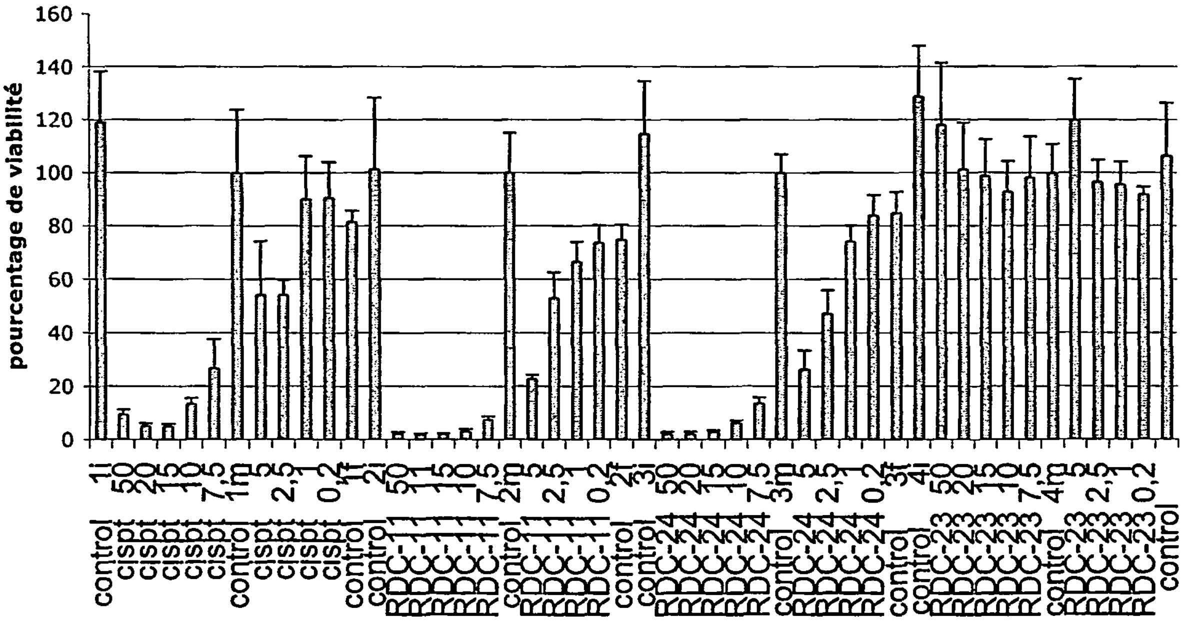


FIGURE 11

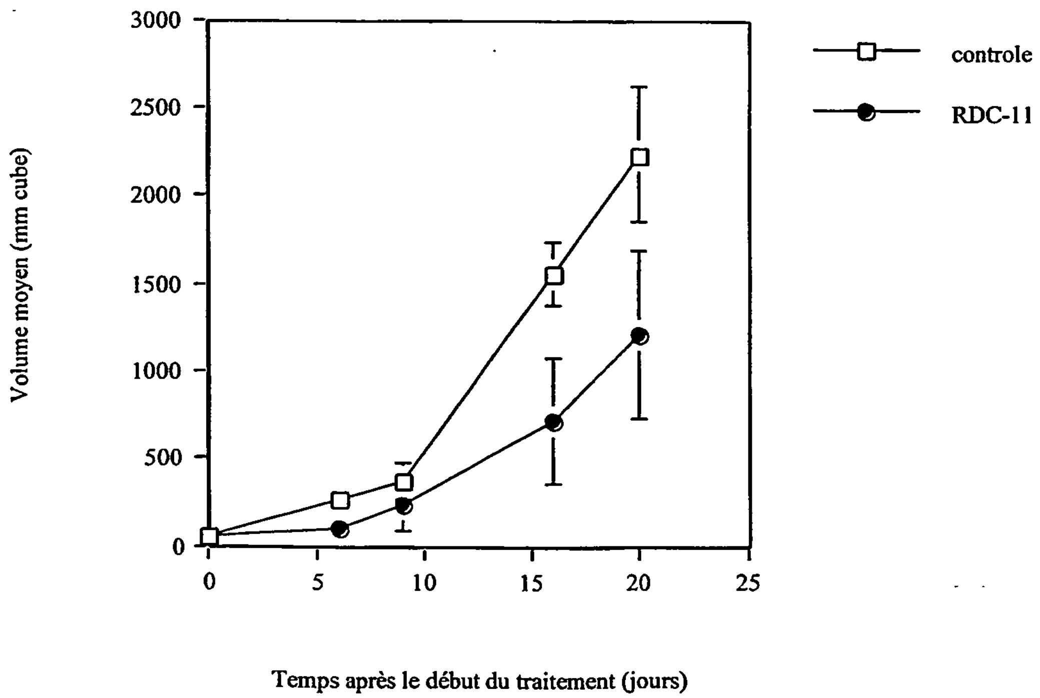
3000
2500
2000
1500
1000
500
0
0
5
10
15
20
25
Volume moyen (mm cube)
Temps après le début du traitement (jours)
controle
RDC-11

FIGURE 12

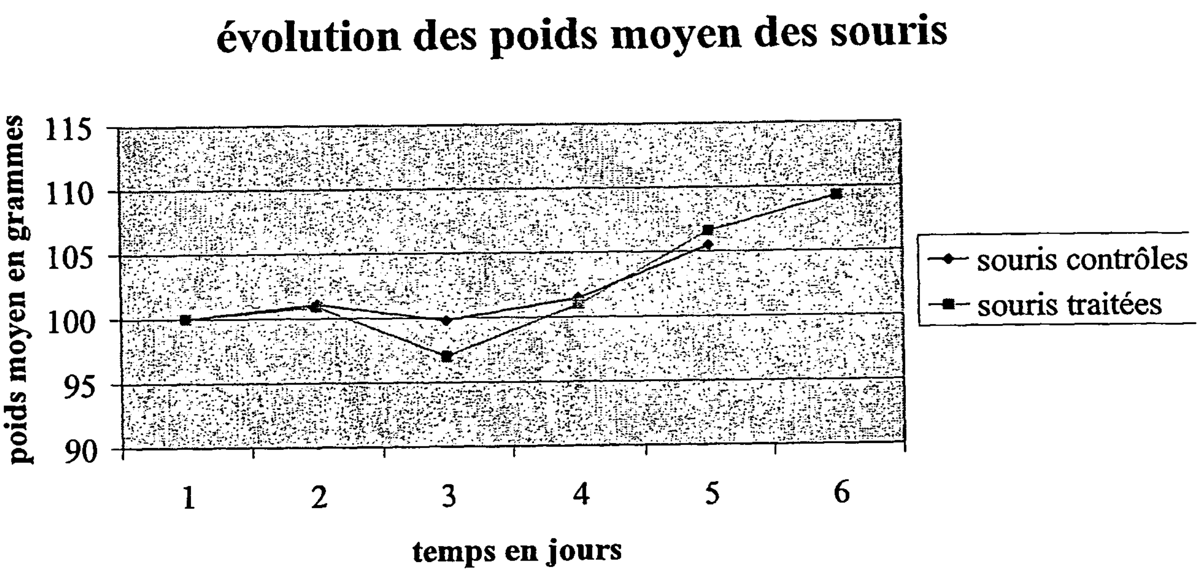
évolution des poids moyen des souris
poids moyen en grammes
115
110
105
100
95
90
1
2
3
4
5
6
temps en jours
souris contrôles
souris traitées

FIGURE 13

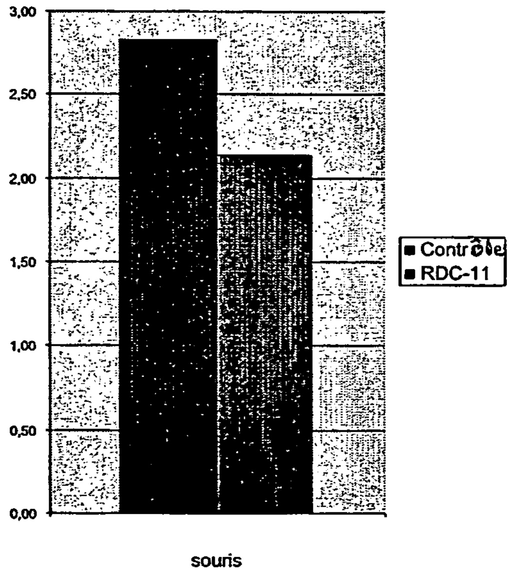


FIGURE 14

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9736595 A
- WO 0056743 A


### Littérature non-brevet citée dans la description


- *Organometallics,* 2002, vol. 21, 5437-5438
- *Organometallics,* 2002, vol. 21, 1184-1189
- **A. D. Ryabov.** *Chem. Rev.,* 1990, vol. 90, 403-424
- *Organometallics,* 1999, vol. 18, 2390-2394
- *J. Organometal. Chem.,* 1995, vol. 494, 187-193
- *Inorg. Chim. Acta,* 1996, vol. 249, 63-67
- *Bull. Soc. Chim. Fr.,* 1997, vol. 134, 947-954
- *Organometallics,* 2003, vol. 22, 347-354
- **Alarcon-Vargas D. ; Ronai Z.** p53-Mdm2--the affair that never ends. *Carcinogenesis,* 2002, vol. 23 (4), 541-7
- **Coelho D ; Fischer B ; Holl V ; Jung GM ; Dufour P ; Bergerat JP ; Denis JM ; Gueulette J ; Bischoff P.** involvement of tp53 in apoptosis induced in human lymphoblastoid cells by fast neutrons. *Radiat. Res.,* 2002, vol. 157 (4), 446-52
- **Katano, K. ; R Safaei et al.** The copper export pump ATP7B modulates the cellular pharmacology of carboplatin in ovarian carcinoma cells. *Mol Pharmacol,* 2003, vol. 64 (2), 466-73
- **Marin, M. C. ; W. G. Kaelin, Jr.** p63 and p73: old members of a new family. *Biochim Biophys Acta,* 2000, vol. 1470 (3), M93-M100
- **Oberlies, N. H. ; D. J. Kroll.** Camptothecin and taxol: historic achievements in natural products research. *J Nat Prod,* 2004, vol. 67 (2), 129-35
- **Prives, C. ; P. A. Hall.** The p53 pathway. *J Pathol,* 1999, vol. 187 (1), 112-26
- **Safaei, R ; K. Katano et al.** Cross-resistance to cisplatin in cells with acquired resistance to copper. *Cancer Chemother Pharmacol,* 2004, vol. 53 (3), 239-46
- **Siddik, Z. H.** Cisplatin: mode of cytotoxic action and molecular basis of resistance. *Oncogene,* 2003, vol. 22 (47), 7265-79
- **Smith, A. L. ; K. C. Nicolaou.** The enediyne antibiotics. *J Med Chem,* 1996, vol. 39 (11), 2103-17
- **Vargas, D. A. ; S. Takahashi et al.** Mdm2: A regulator of cell growth and death. *Adv Cancer Res,* 2003, vol. 89, 1-34
- **Yang, Y. ; C. C. Li et al.** Regulating the p53 system through ubiquitination. *Oncogene,* 2004, vol. 23 (11), 2096-106